# EUROPÄISCHE PATENTSCHRIFT

(11) **EP 1 105 368 B1**
(45) Veröffentlichungstag und Bekanntmachung des Hinweises auf die Patenterteilung: **30.10.2002**
(21) Anmeldenummer: 99942830.3
(22) Anmeldetag: 10.08.1999
(51) Int. Cl.: C07C 211/63, G03F 7/027, G03F 7/004, C07D 471/04, C07D 471/08

(54) **PHOTOAKTIVIERBARE STICKSTOFFHALTIGE BASEN**
PHOTOACTIVATABLE BASES CONTAINING NITROGEN
BASES PHOTO-ACTIVABLES RENFERMANT DE L'AZOTE

(30) Priorität: 21.08.1998 EP 98810819
(43) Veröffentlichungstag der Anmeldung: 13.06.2001
(73) Patentinhaber: Ciba Specialty Chemicals Holding Inc., 4057 Basel (CH)
(72) Erfinder: BAUDIN, Gisèle, CH-4123 Allschwil (CH); TURNER, Sean, Colm, Evanston, IL 60201 (US); CUNNINGHAM, Allan, Francis, Somers, NY 10589 (US)
(86) Internationale Anmeldenummer: EP9905790
(87) Internationale Veröffentlichungsnummer: WO00010964

(56) Entgegenhaltungen:
- EP-A- 0 223 587
- EP-A- 0 367 164
- EP-A- 0 548 826
- EP-A- 0 775 706
- GB-A- 2 307 474
- US-A- 3 539 632

## Beschreibung

Die Erfindung betrifft α-Amonium-, Iminium- oder Amidiniumalkene in der Form ihrer Tetraaryl- oder Triarylalkylborat-Salze, ein Verfahren zu deren Herstellung, sowie zu deren photochemischer Spaltung unter Bildung eines Amins, Imins oder Amidins und ihrer Verwendung in basenkatalysiert vernetzbaren Systemen oder Hybridsystemen (sowohl radikalisch als auch basenkatalysiert polymerisierbare oder vernetzbare Zusammensetzungen).

α-Ammoniumalken-Salze, insbesondere 2-Arylallylammonium-Salze sowie Di-allylamm-oniumverbindungen, sind bereits beispielsweise in US 3539632, J. Org. Chem. 52 (16) (1987), 3683, EP-A 0548826 oder in J. Am. Chem. Soc. 101 (11) (1979), 3097, beschrieben. Die GB-A 2307474 offenbart Organoborat-Photoinitiatoren, welche u.a. Ammoniumkationen enthalten.

Neben radikalisch polymerisierbaren Oligomeren oder Monomeren sind vor allem für photolithographische Verfahren basisch katalysierbare Systeme bekannt geworden. Diese Systeme benötigen einen Photoinitiator, der bei der Belichtung eine Base abspaltet. R. Mac-Kean et al., Polym. Mater. Sci. Eng. (1992), 66, 237-238 berichten beispielsweise über Photostrukturierung von Polyimid, wobei als Photoinitiatoren spezifische Carbamate eingesetzt werden.

Die photolytische Generation von Basen und Photopolymerisationsreaktionen mit diesen Basen sind auch beschrieben, wobei Gebrauch gemacht wird von verschiedenen Typen von photolabilen Verbindungen, z.B. Carbamaten (Cameron et al., US Patent 5545509 und darin zitierte Referenzen; Cameron und Frechet, J. Am. Chem. Soc. (1991) 113, 4303), α-Keto-Carbamate (Cameron et al., J. Am. Chem. Soc. (1996) 118, 12925), O-Acyloxime (Tsunooka et al., J. Polymer Sci.: Part A: Polymer Chem. (1994), 32, 2177), Formamide (Nishikubo et al., Polym. J. (1993) 25, 421; idem, J. Polymer Sci.: Part A: Polymer Chem. (1993), 31, 3013) und Co-Amin-Komplexe (C. Kutal et al. J. Electrochem. Soc. (1987), 134, 2280).

Es wurde nun überraschend gefunden, dass bestimmte α-Ammonium-, Iminium- oder Amidiniumalkene in Form ihrer Tetraaryl- oder Triarylalkylboratsalze bei der Belichtung mit sichtbarem Licht oder UV-Licht eine Amin-, Imin- oder Amidingruppe abspalten. Diese Gruppen sind genügend basisch, um eine Vielzahl von basisch katalysierbaren Reaktionen, insbesondere Polymerisationsreaktionen, auszulösen. Die Verbindungen weisen eine hohe Empfindlichkeit auf und je nach Wahl des Substitutionsmusters kann das Absorptionsspektrum in einem weiten Bereich variiert werden.

Die Verbindungen ermöglichen es, sogenannte Eintopfsysteme mit basisch katalysierbaren Oligomeren oder Monomeren herzustellen, die eine ausserordentlich hohe Lagerstabilität aufweisen. Erst nach der Belichtung wird beispielsweise eine Polymerisation ausgelöst. Die Systeme können vollständig oder weitgehend lösungsmittelfrei formuliert werden, da die Verbindungen in den Monomeren oder Oligomeren ohne Beeinflussung gelöst werden können. Der aktive Katalysator entsteht erst nach der Belichtung. Diese Systeme können für viele Zwecke eingesetzt werden, wie zum Beispiel für Lackierungen, Beschichtungen, Formmassen oder photolithographische Abbildungen.

Ein Gegenstand der Erfindung sind Verbindungen der Formel (I) 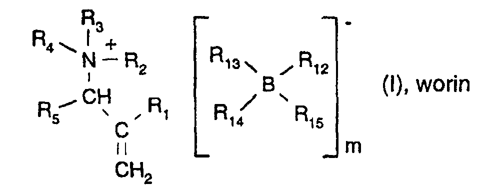
m 1 oder 2 ist und der Anzahl positiver Ladungen des Kations entspricht;
R₁ Phenyl, Naphthyl, Phenanthryl, Anthracyl, Pyrenyl, 5,6,7,8-Tetrahydro-2-naphthyl, 5,6,7,8-Tetrahydro-1-naphthyl, Thienyl, Benzo[b]thienyl, Naphto[2,3-b]thienyl, Thianthrenyl, Dibenzofuryl, Chromenyl, Xanthenyl, Thioxanthyl, Phenoxathiinyl, Pyrrolyl, Imidazolyl, Pyrazolyl, Pyrazinyl, Pyrimidinyl, Pyridazinyl, Indolizinyl, Isoindolyl, Indolyl, Indazolyl, Purinyl, Chinolizinyl, Isochinolyl, Chinolyl, Phthalazinyl, Naphthyridinyl, Chinoxalinyl, Chinazolinyl, Cinnolinyl, Pteridinyl, Carbazolyl, β-Carbolinyl, Phenanthridinyl, Acridinyl, Perimidinyl, Phenanthrolinyl, Phenazinyl, Isothiazolyl, Phenothiazinyl, Isoxazolyl, Furazanyl, Terphenyl, Stilbenyl, Fluorenyl oder Phenoxazinyl darstellt, wobei diese Reste unsubstituiert oder mit C₁-C₁₈-Alkyl, C₃-C₁₈-Alkenyl, C₃-C₁₈-Alkinyl, C₁-C₁₈-Halogenalkyl, NO₂, NR₆R₇, N₃, OH, CN, OR₈, SR₈, C(O)R₉, C(O)OR₁₀ oder Halogen ein- oder mehrfach substituiert sind, oder R₁ einen Rest der Formeln A oder B 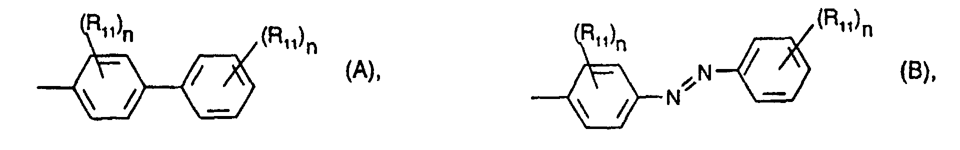 bedeutet;
R₂, R₃ und R₄ unabhängig voneinander Wasserstoff, C₁-C₁₈-Alkyl, C₃-C₁₈-Alkenyl, C₃-C₁₈-Alkinyl oder Phenyl sind oder unabhängig voneinander R₂ und R₃ und/oder R₄ und R₃ eine C₂-C₁₂-Alkylenbrücke bilden; oder R₂, R₃, R₄ mit dem Stickstoffatom, an welches sie gebunden sind, eine Phosphazen-Base vom Typ P₁, P₂, P₄ oder eine Gruppe der Strukturformeln (a), (b), (c), (d), (e), (f) oder (g) bilden 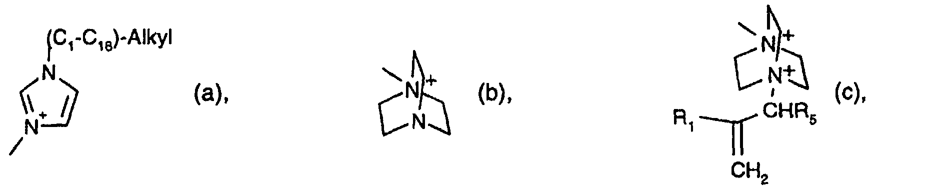 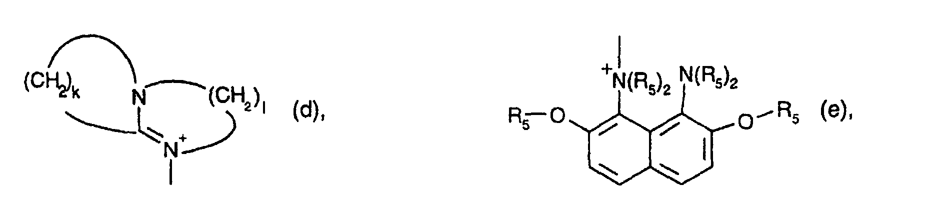 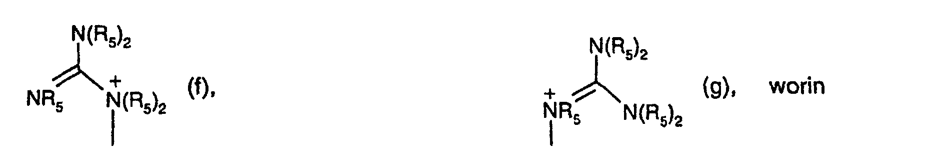
k und I unabhängig voneinander eine Zahl von 2 bis 12 bedeuten;
R₅, R₆, R₇, R₈, R₉ und R₁₀ unabhängig voneinander Wasserstoff oder C₁-C₁₈-Alkyl sind;
R₁₁ C₁-C₁₈-Alkyl, C₂-C₁₈-Alkenyl, C₂-C₁₈-Alkinyl, C₁-C₁₈-Halogenalkyl, NO₂, NR₆R₇, OH, CN, OR₈, SR₈, C(O)R₉, C(O)OR₁₀ oder Halogen ist; und
n 0 oder eine Zahl 1, 2 oder 3 ist;
R₁₂, R₁₃ und R₁₄ für Phenyl oder einen anderen aromatischen Kohlenwasserstoff stehen, wobei diese Reste unsubstituiert oder mit C₁-C₁₈-Alkyl, C₃-C₁₈-Alkenyl, C₃-C₁₈-Alkinyl, C₁-C₁₈-Halogenalkyl, NO₂, OH, CN, OR₈, SR₈, C(O)R₉, C(O)OR₁₀ oder Halogen ein- oder mehrfach substituiert sind;
R₁₅ C₁-C₁₈-Alkyl, Phenyl oder einen anderen aromatischen Kohlenwasserstoff darstellt, wobei die Reste Phenyl und aromatischer Kohlenwasserstoff unsubstituiert oder mit C₁-C₁₈-Alkyl, C₃-C₁₈-Alkenyl, C₃-C₁₈-Alkinyl, C₁-C₁₈-Halogenalkyl, NO₂, OH, CN, OR₈, SR₈, C(O)R₉, C(O)OR₁₀ oder Halogen ein- oder mehrfach substituiert sind, oder R₁₅ für einen Rest 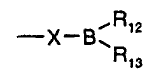 steht; und
X C₁-C₂₀-Alkylen, C₂-C₂₀-Alkylen, welches durch -O-, -S- oder NR₈ unterbrochen ist,
   bedeutet oder X 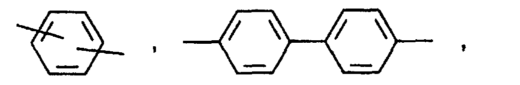 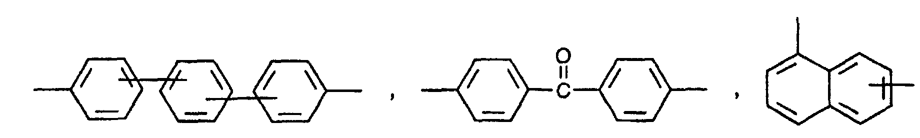 oder 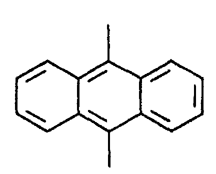 ist.

Durch die Wahl des aromatischen oder heteroaromatischen Rest R₁ sowie des jeweiligen Borat-Anions lässt sich das Maximum der Absorption in einem weiten Bereich variieren und damit die Photoempfindlichkeit der Verbindungen vom UV bis in den Tageslichtbereich hinein verschieben.

Alkyl in den verschiedenen Resten mit bis zu 18 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl.
Bevorzugt ist Alkyl mit 1 bis 12 oder 1 bis 8, insbesondere 1 bis 6 oder 1 bis 4 Kohlenstoffatomen.

Alkenyl mit 3 bis 18 Kohlenstoffatomen bedeutet einen verzweigten oder unverzweigten Rest wie beispielsweise Propenyl, 2-Butenyl, 3-Butenyl, Isobutenyl, n-2,4-Pentadienyl, 3-Methyl-2-butenyl, n-2-Octenyl, n-2-Dodecenyl, iso-Dodecenyl, Oleyl, n-2-Octadecenyl oder n-4-Octadecenyl.
Bevorzugt ist Alkenyl mit 3 bis 12, insbesondere 3 bis 6 Kohlenstoffatomen.

Alkinyl mit 3 bis 18 Kohlenstoffatomen bedeutet einen verzweigten oder unverzweigten Rest wie beispielsweise Propinyl ( ―CH₂-C≡CH ), 2-Butinyl, 3-Butinyl, n-2-Octinyl, oder n-2-Octadecinyl. Bevorzugt ist Alkinyl mit 3 bis 12, insbesondere 3 bis 6 Kohlenstoffatomen.

Die C₂-C₁₂-Alkylenbrücke bedeutet Ethylen, Propylen, Butylen, Pentylen, Hexylen, Heptylen Octylen, Nonylen, Decylen, Undecylen oder Dodecylen.

Halogen bedeutet Fluor, Chlor, Brom oder lod.

Beispiele für C₁-C₁₈-Halogenalkyl sind ganz oder teilweise halogeniertes C₁-C₁₈-Alkyl. Beispiele hierfür sind die Stellungsisomeren von Mono- bis Decafluorpentyl, Mono- bis Octafluorbutyl, Mono- bis Hexafluorpropyl, Mono- bis Tetrafluorethyl sowie Mono- und Difluormethyl sowie die entsprechenden Chlor, Brom und lod Verbindungen. Bevorzugt sind die perfluorierten Alkylreste. Beispiele hierfür sind Perfluorpentyl, Perfluorbutyl, Perfluorpropyl, Perfluorethyl und insbesondere Trifluormethyl.

Beispiele für die NR₆R₇ Aminogruppe sind die jeweiligen Monoalkyl- oder Dialkylaminogruppen wie Methylamino, Ethylamino, Propylamino, Butylamino, Pentylamino, Hexylamino, Octadecylamino, Dimethylamino, Diethylamino, Dipropylamino, Diisopropylamino, Di-n-butylamino Di-isobutylamino, Dipentylamino, Dihexylamino oder Dioctadecylamino. Weitere Dialkylaminogruppen sind solche, bei denen die beiden Reste unabhängig voneinander verzweigt oder unverzweigt sind wie beispielsweise Methylethylamino, Methyl-n-propylamino, Methylisopropylamino, Methyl-n-butylamino, Methylisobutylamino, Ethylisopropylamino, Ethyl-n-butylamino, Ethylisobutylamino, Ethyl-tert-butylamino, Isopropyl-n-butylamino oder Isopropylisobutylamino.

Die Alkoxygruppe OR₈ mit bis zu 18 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy.

Bevorzugt ist Alkoxy mit 1 bis 12, insbesondere 1 bis 8, z.B. 1 bis 6 oder 1 bis 4 Kohlenstoffatomen.

Beispiele für die Thioalkylgruppe SR₈ sind Thiomethyl, Thioethyl, Thiopropyl, Thiobutyl, Thiopentyl, Thiohexyl, Thioheptyl, Thiooctyl oder Thiooctadecyl, wobei die Alkylreste linear oder verzweigt sein können.

Aromatische Kohlenwasserstoffe, wie sie z.B. in den erfindungsgemässen Verbindungen (R₁₂, R₁₃, R₁₄ oder R₁₅) enthalten sein können, können z.B. ein oder mehrere, insbesondere ein oder zwei, Heteroatome enthalten. Als Heteroatome kommen z.B. N, O, P oder S, bevorzugt N oder O, in Frage. Beispiele für aromatische Kohlenwasserstoffe sind: Phenyl, α-und β-Naphthyl, Stilbenyl, Biphenyl, o-, m-, p-Terphenyl, Triphenylphenyl, Binaphthyl, Anthracyl, Phenanthryl, Pyrenyl, Furan-2-yl oder Furan-3-yl, Thiophen-2-yl oder Thiophen-3-yl, Pyridin-2-yl, Pyridin-3-yl oder Pyridin-4-yl, Chinolyl oder Isochinolyl.

Beispiele für den Rest R₁ sind Phenyl, Naphthyl, Phenanthryl, Anthracyl, Pyrenyl, 5,6,7,8-Tetrahydro-2-naphthyl, 5,6,7,8-Tetrahydro-1-naphthyl, Thienyl, Benzo[b]thienyl, Naphto[2,3-b]-thienyl, Thiathrenyl, Dibenzofuryl, Chromenyl, Xanthenyl, Phenoxathiinyl, Pyrrolyl, Imidazolyl, Pyrazolyl, Pyrazinyl, Pyrimidinyl, Pyridazinyl, Indolizinyl, Isoindolyl, Indolyl, Indazolyl, Purinyl, Chinolizinyl, Isochinolyl, Chinolyl, Phthalazinyl, Naphthyridinyl, Chinoxalinyl, Chinazolinyl, Cinnolinyl, Pteridinyl, Carbazolyl, β-Carbolinyl, Phenanthridinyl, Acridinyl, Perimidinyl, Phenanthrolinyl, Phenazinyl, Isothiazolyl, Phenothiazinyl, Isoxazolyl, Furazanyl, Biphenyl, Stilbenyl, Terphenyl, Fluorenyl, Phenoxazinyl, Methoxyphenyl, 2,4-Dimethoxyphenyl, 2,4,6-Trimethoxyphenyl, Bromphenyl, Toluyl, Xylyl, Mesityl, Nitrophenyl, Dimethylaminophenyl, Diethylaminophenyl, Aminophenyl, Diamionophenyl, 1-Naphthyl, 2-Naphthyl, 1-Phenylamino-4-naphthyl, 1-Methylnaphthyl, 2-Methylnaphthyl, 1-Methoxy-2-naphthyl, 2-Methoxy-1-naphthyl, 1-Dimethylamino-2-naphthyl, 1,2-Dimethyl-4-naphthyl, 1,2-Dimethyl-6-naphthyl, 1,2-Dimethyl-7-naphthyl, 1,3-Dimethyl-6-naphthyl, 1,4-Dimethyl-6-naphthyl, 1,5-Dimethyl-2-naphthyl, 1,6-Dimethyl-2-naphthyl, 1-Hydroxy-2-naphthyl, 2-Hydroxy-1-naphthyl, 1,4-Dihydroxy-2-naphthyl, 7-Phenanthryl, 1-Anthryl, 2-Anthryl, 9-Anthryl, 3-Benzo[b]thienyl, 5-Benzo[b]thienyl, 2-Benzo[b]thienyl, 4-Dibenzofuryl, 4,7-Dibenzofuryl, 4-Methyl-7-dibenzofuryl, 2-Xanthenyl, 8-Methyl-2-xanthenyl, 3-Xanthenyl, 2-Phenoxathiinyl, 2,7-Phenoxathiinyl, 2-Pyrrolyl, 3-Pyrrolyl, 5-Methyl-3-pyrrolyl, 2-Imidazolyl, 4-Imidazolyl, 5-Imidazolyl, 2-Methyl-4-imidazolyl, 2-Ethyl-4-imidazolyl, 2-Ethyl-5-imidazolyl, 3-Pyrazolyl, 1-Methyl-3-pyrazolyl, 1-Propyl-4-pyrazolyl, 2-Pyrazinyl, 5,6-Dimethyl-2-pyrazinyl, 2-Indolizinyl, 2-Methyl-3-isoindolyl, 2-Methyl-1-isoindolyl, 1-Methyl-2-indolyl, 1-Methyl-3-indolyl, 1,5-Dimethyl-2-indolyl, 1-Methyl-3-indazolyl, 2,7-dimethyl-8-purinyl, 2-Methoxy-7-methyl-8-purinyl, 2-Chinolizinyl, 3-Isochinolyl, 6-Isochinolyl, 7-Isochinolyl, Isochinolyl, 3-Methoxy-6-isochinolyl, 2-Chinolyl, 6-Chinolyl, 7-Chinolyl, 2-Methoxy-3-chinolyl, 2-Methoxy-6-chinolyl, 6-Phthalazinyl, 7-Phthalazinyl, 1-Methoxy-6-phthalazinyl, 1,4-Dimethoxy-6-phthalazinyl, 1,8-Naphthyridin-2-yl, 2-Chinoxalinyl, 6-Chinoxalinyl, 2,3-Dimethyl-6-chinoxalinyl, 2,3-Dimethoxy-6-chinoxalinyl, 2-Chinazolinyl, 7-Chinazolinyl, 2-Dimethylamino-6-chinazolinyl, 3-Cinnolinyl,6- Cinnolinyl, 7-Cinnolinyl, 3-Methoxy-7-cinnolinyl, 2-Pteridinyl, 6-Pteridinyl, 7-Pteridinyl, 6,7-Dimethoxy-2-pteridinyl, 2-Carbazolyl, 3-Carbazolyl, 9-Methyl-2-Carbazolyl, 9-Methyl-3-Carbazolyl, β-Carbolin-3-yl, 1-Methyl-β-carbolin-3-yl, 1-Methyl-β-Carbolin-6-yl, 3-Phenanthridinyl, 2-Acridinyl, 3-Acridinyl, 2-Perimidinyl, 1-Methyl-5-perimidinyl, 5-Phenanthrolinyl, 6-Phenanthrolinyl, 1-Phenazinyl, 2-Phenazinyl, 3-Isothiazolyl, 4-Isothiazolyl, 5-Isothiazolyl, 2-Phenothiazinyl, 3-Phenothiazinyl, 10-Methyl-3-phenothiazinyl, 3-Isoxazolyl, 4-Isoxazolyl, 5-Isoxazolyl, 4-Methyl-3-furazanyl, 2-Phenoxazinyl oder 10-Methyl-2-phenoxazinyl.

Beispiele für Phosphazen-Basen vom Typ P₁, P₂ oder P₄ sind 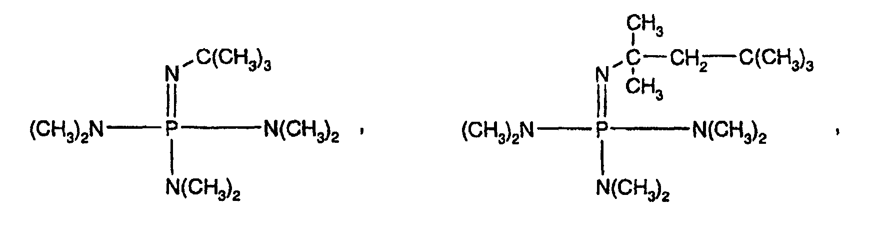 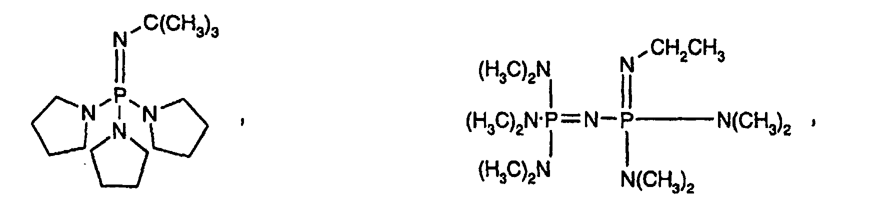 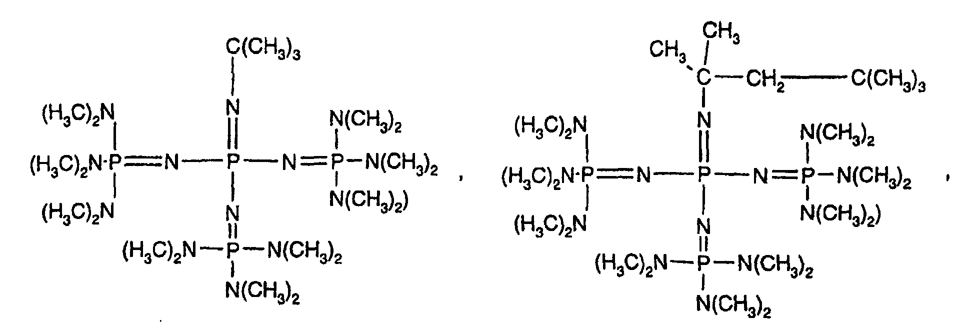 wobei die Phosphazenbasen sowohl über den Iminstickstoff als auch über eines der tertiären Stickstoffatome an die CH₂-Gruppe des Alkens gebunden sein können.
Bevorzugt sind sie über eines der tertiären Stickstoffatome an die CH₂-Gruppe des Ketons gebunden.
Phosphazenbasen vom Typ P₁, P₂ oder P₄ sind dem Fachmann bekannt und werden z.B in Chemikalienkatalogen angeboten. Beispiele für diese Nomenklatur sind ausserdem in Angew. Chem. 1993, 105, 1420 beschrieben.

Bevorzugt bedeutet R₁ Phenyl, Naphthyl, Phenanthryl, Anthracyl, Pyrenyl, 5,6,7,8-Tetrahydro-2-naphthyl, 5,6,7,8-Tetrahydro-1-naphthyl, Thienyl, Benzo[b]thienyl, Naphto[2,3-b]thienyl, Thiathrenyl, Dibenzofuryl, Chromenyl, Xanthenyl, Thioxanthyl, Phenoxathiinyl, Pyrrolyl, Imidazolyl, Pyrazolyl, Pyrazinyl, Pyrimidinyl, Pyridazinyl, Indolizinyl, Isoindolyl, Indolyl, Indazolyl, Purinyl, Chinolizinyl, Isochinolyl, Chinolyl, Phthalazinyl, Naphthyridinyl, Chinoxalinyl, Chinazolinyl, Cinnolinyl, Pteridinyl, Carbazolyl, β-Carbolinyl, Phenanthridinyl, Acridinyl, Perimidinyl, Phenanthrolinyl, Phenazinyl, Isothiazolyl, Phenothiazinyl, Isoxazolyl, Furazanyl, Terphenyl, Stilbenyl, Fluorenyl oder Phenoxazinyl, wobei diese Reste unsubstituiert oder mit C₁-C₁₈-Alkyl, C₃-C₁₈-Alkenyl, C₃-C₁₈-Alkinyl, C₁-C₁₈-Halogenalkyl, NO₂, NR₆R₇, N₃, OH, CN, OR₈, SR₈, C(O)R₉, C(O)OR₁₀ oder Halogen ein- oder mehrfach substituiert sind; oder R₁ einen Rest der Formeln A oder B 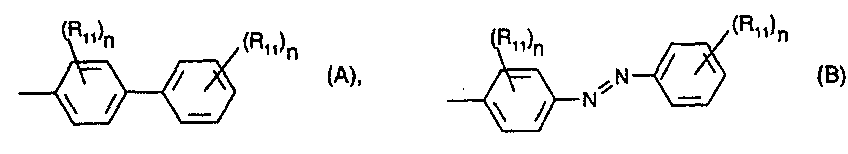 darstellt.

Besonders bevorzugt bedeutet R₁ Phenyl, Naphthyl, Pyrenyl, Thioxanthyl oder Phenothiazinyl, wobei diese Reste unsubstituiert oder mit C₁-C₁₈-Alkyl, C₁-C₁₈-Halogenalkyl, NR₆R₇, CN, NO₂, SR₈ oder OR₈ ein- oder mehrfach substituiert sind.

Bevorzugt bedeuten R₂, R₃ und R₄ unabhängig voneinander Wasserstoff, C₁-C₁₈-Alkyl oder es bilden unabhängig voneinander R₂ und R₃ und/oder R₄ und R₃ eine C₂-C₁₂-Alkylenbrücke; oder R₂, R₃, R₄ bilden mit dem Stickstoffatom, an welches sie gebunden sind, eine Gruppe der Strukturformeln (a), (b), (c), (d), (e), (f), (g), wie vorstehend angegeben, oder eine Phosphazen-Base vom Typ P₁, P₂ oder P₄.
k und I bedeuten unabhängig voneinander eine Zahl von 2 bis 12, bevorzugt bedeuten sie eine Zahl von 2 bis 6.

Besonders bevorzugt sind Verbindungen, worin R₂, R₃ und R₄ unabhängig voneinander C₁-C₁₈-Alkyl sind oder R₂, R₃, R₄ mit dem Stickstoffatom eine Gruppe der Strukturformeln (a), (b), (c), (d) oder (e), wie vorstehend angegeben, bilden.

Bevorzugt bedeuten R₁₂, R₁₃, R₁₄ Phenyl, Biphenyl, Naphthyl, Anthracyl oder Phenanthryl, wobei diese Reste unsubstituiert oder mit C₁-C₁₈-Alkyl, C₁-C₁₈-Halogenalkyl, NO₂, OH, CN, OR₈, oder Halogen ein- oder mehrfach substituiert sind und R₁₅ bedeutet C₁-C₁₈-Alkyl oder unsubstituiertes oder mit C₁-C₁₈-Alkyl, C₁-C₁₈-Halogenalkyl, NO₂, OH, CN, OR₈ oder Halogen ein- oder mehrfach substituiertes Phenyl.

Geeignete Boratanionen zum Stickstoffbasenkation in den Verbindungen der Formel I sind beispielsweise auch US 4772530, GB 2307474, GB 2307473, GB 2307472, EP 775706 zu entnehmen. Beispiele sind Triphenylbutylborat, Triphenylhexylborat, Triphenylmethylborat, Dimesityl-phenyl-methyl- oder -butylborat, Di(bromomesityl)-phenyl-methyl- oder-butylborat, Tris(3-fluorphenyl)-hexylborat, Tris(3-fluorphenyl)-methyl- oder -butylborat, Dichloromesitylphenyl-methyl- oder -butylborat, Tris(dichloromesityl)-methylborat, Tris(3-chtorphenyl)-hexylborat, Tris(3-chlorphenyl)-methyl- oder -butylborat, Tris(3-bromphenyl)-hexylborat, Tris-(3-bromphenyl)-methyl- oder -butylborat, Tris(3,5-difluorphenyl)-hexylborat, Dimesityl-biphenyl-butylborat, Dimesityl-naphthylmethyl- oder -butylborat, Di(o-tolyl)-9-anthracyl-methyloder -butylborat, Dimesityl-9-phenanthryl-phenyl- oder -butylborat oder

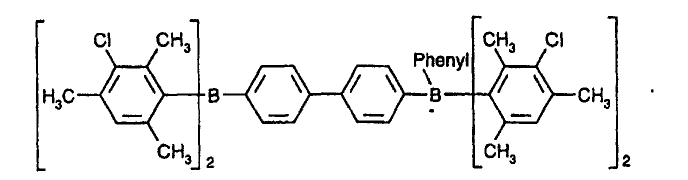

Die Herstellung dieser Anionen ist in den vorstehend genannten Publikationen beschrieben.

Die Herstellung der Bromide oder Jodide der erfindungsgemässen Verbindungen der Formel I (=Formel la, bzw. la') erfolgt beispielsweise durch Umsetzung eines Amins der Formel II mit einem α-Halogenalken der Formel III

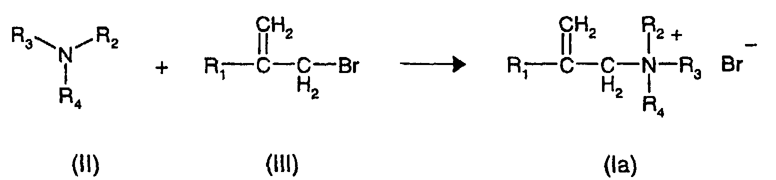

Die Herstellung der Halogenide kann beispielsweise auch durch Alkylierung eines aromatisch substituierten α-Aminoalkens erfolgen:

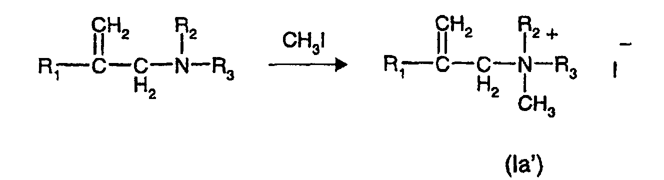

Aus den entprechenden Halogeniden der erfindungsgemässen Verbindungen lassen sich durch Anionenaustauschreaktionen die jeweiligen Borate erhalten. Die Reaktion kann in an sich bekannter Weise durchgeführt werden. Vorteilhaft wird ein Lösungsmittel oder Gemisch von Lösungsmitteln mitverwendet, zum Beispiel Wasser, Kohlenwasserstoffe (Benzol, Toluol, Xylol), halogenierte Kohlenwasserstoffe (Methylenchlorid, Chloroform, Tetrachlorkohlenstoff, Chlorbenzol), Alkanole (Methanol, Ethanol, Ethylenglykolmonomethylether), und Ether (Diethylether, Dibutylether, Ethylenglykoldimethylether) oder Gemische davon.

Die Reaktion wird zweckmässig in einem Temperaturbereich von -10°C bis + 100°C durchgeführt. Bevorzugt wird sie bei 10°C bis 50°C durchgeführt.

Ebenfalls Gegenstand der Erfindung ist ein Verfahren zur Herstellung der Verbindungen der Formel I, wobei in einem ersten Schritt eine Stickstoffbase der Formel II

NR₂R₃R₄ (II)

mit einem α- Halogenalken der Formel III 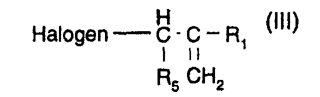 zu einer Verbindung der Formel IV 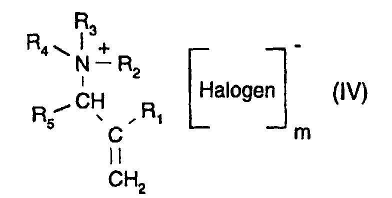 umgesetzt wird und in einem zweiten Schritt die Verbindungen der Formel IV mit einer Verbindung der Formel V 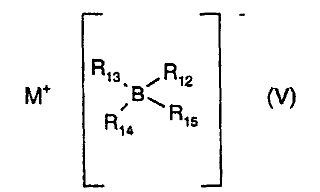 zur Verbindung der Formel I umgesetzt wird, worin
Halogen Brom oder lod bedeutet und
M Na, K oder Ammonium ist, und die Reste
R₁, R₂, R₃, R₄, R₅, R₁₂, R₁₃, R₁₄ und R₁₅ die vorstehend angegeben Bedeutungen einschliesslich der Bevorzugungen haben.

Ein weiterer Gegenstand der Erfindung ist eine Zusammensetzung, enthaltend
A) mindestens eine Verbindung der Formel (I), wie vorstehend beschrieben, und
B) mindestens eine organische Verbindung, welche zu einer basenkatalysierten Additions-oder Substitutionsreaktion befähigt ist.

Als Komponente B) in den erfindungsgemässen Zusammensetzungen kommen beispielsweise Epoxysyteme in Frage. Für die Herstellung von erfindungsgemässen härtbaren Gemischen mit Epoxidharzen als Komponente B) eignen sich die in der Epoxidharztechnik üblichen Epoxidharze. Beispiele für solche Epoxidharze sind:
I) Polyglycidyl- und Poly-(β-methylglycidyl)-ester, erhältlich durch Umsetzung einer Verbindung mit mindestens zwei Carboxylgruppen im Molekül und Epichlorhydrin bzw. β-Methylepichlorhydrin. Die Umsetzung erfolgt zweckmässig in der Gegenwart von Basen.
   Als Verbindung mit mindestens zwei Carboxylgruppen im Molekül können aliphatische Polycarbonsäuren verwendet werden. Beispiele für solche Polycarbonsäuren sind Oxalsäure, Bernsteinsäure, Glutarsäure, Adipinsäure, Pimelinsäure, Korksäure, Azelainsäure oder dimerisierte bzw. trimerisierte Linolsäure. Es können aber auch cycloaliphatische Polycarbonsäuren eingesetzt werden, wie beispielsweise Tetrahydrophthalsäure, 4-Methyltetrahydrophthalsäure, Hexahydrophthalsäure oder 4-Methylhexahydrophthalsäure. Weiterhin können aromatische Polycarbonsäuren Verwendung finden, wie beispielsweise Phthalsäure, Isophthalsäure oder Terephthalsäure.
II) Polyglycidyl-oder Poly-(β-methylglycidyl)-ether, erhältlich durch Umsetzung einer Verbindung mit mindestens zwei freien alkoholischen Hydroxygruppen und/oder phenolischen Hydroxygruppen und Epichlorhydrin oder β-Methylepichlorhydrin unter alkalischen Bedingungen, oder in Anwesenheit eines sauren Katalysators und anschliessende Alkalibehandlung.
   Die Glycidylether dieses Typs leiten sich beispielsweise von acyclischen Alkoholen ab, wie von Ethylenglykol, Diethylenglykol und höheren Poly-(oxyethylen)-glykolen, Propan-1,2-diol oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Pentaerythrit, Sorbit, sowie von Polyepichlorhydrinen. Sie leiten sich aber auch beispielsweise von cycloaliphatischen Alkoholen, wie 1,4-Cyclohexandimethanol, Bis-(4-hydroxycyclohexyl)-methan oder 2,2-Bis-(4-hydroxycyclohexyl)-propan, ab oder sie besitzen aromatische Kerne, wie N,N-Bis-(2-hydroxyethyl)-anilin oder p,p'-Bis-(2-hydroxyethylamino)-diphenylmethan. Die Glycidylether können sich auch von einkerningen Phenolen ableiten, wie beispielsweise von Resorcin oder Hydrochinon, oder sie basieren auf mehrkernigen Phenolen, wie beispielsweise Bis-(4-hydroxyphenyl)-methan, 4,4'-Dihydroxybiphenyl, Bis-(4-hydroxyphenyl)-sulfon, 1,1,2,2-Tetrakis-(4-hydroxyphenyl)-ethan, 2,2-Bis-(4-hydroxyphenyl)-propan, 2,2-Bis-(3,5-dibrom-4-hydroxyphenyl)-propan sowie von Novolaken, erhältlich durch Kondensation von Aldehyden, wie Formaldehyd, Acetaldehyd, Chloral oder Furfuraldehyd, mit Phenolen, wie Phenol, oder mit Phenolen, die im Kern mit Chloratomen oder C₁-C₉-Alkylgruppen substituiert sind, wie beispielsweise 4-Chlorphenol, 2-Methylphenol, oder 4-tert.-Butylphenol oder durch Kondensation mit Bisphenolen, solche der oben genannten Art.
III) Poly-(N-glycidyl)-verbindungen, erhältlich durch Dehydrochlorierung der Reaktionsprodukte von Epichlorhydrin mit Aminen, die mindestens zwei Aminwasserstoffatome enthalten. Bei diesen Aminen handelt es sich zum Beispiel um Anilin, n-Butylamin, Bis(4-aminophenyl)-methan, m-Xylylendiamin oder Bis-(4-methylaminophenyl)-methan.
   Zu den Poly-(N-glycidyl)-verbindungen zählen aber auch Triglycidylisocyanurat, N,N'-Diglycidylderivate von Cycloalkylenharnstoffen, wie Ethylenharnstoff oder 1,3-Propylenharnstoff, und Diglycidylderivate von Hydantoinen, wie von 5,5-Dimethyhydantoin.
IV) Poly-(S-glycidyl)-verbindungen, beispielsweise Di-S-glycidylderivate, die sich von Dithiolen, wie beispielsweise Ethan-1,2-dithiol oder Bis-(4-mercaptomethylphenyl)-ether ableiten.
V) Cycloaliphatische Epoxidharze, beispielsweise Bis-(2,3-epoxycyclopentyl)-ether, 2,3-Epoxycydopentylglycidylether, 1,2-Bis-(2,3-epoxycyclopentyloxy)-ethan oder 3,4-Epoxycyclohexylmethyl-3',4'-epoxycyclohexancarboxylat.
   Es lassen sich aber auch Epoxidharze verwenden, bei denen die 1,2-Epoxidgruppen an unterschiedliche Heteroatome bzw. funktionelle Gruppen gebunden sind; zu diesen Verbindungen zählen beispielsweise das N,N,O-Triglycidylderivat des 4-Aminophenols, der Glycidylether-glycidylester der Salicylsäure, N-Glycidyl-N'-(2-glycidyloxypropyl)-5,5-dimethylhydantoin oder 2-Glycidyloxy-1,3-bis-(5,5-dimethyl-1-glycidylhydantoin-3-yl)-propan.

Als Komponente B) können auch Gemische von Epoxidharzen verwendet werden.
Erfindungsgemäss ist daher auch eine Zusammensetzung, worin die Komponente B) ein Epoxidharz oder ein Gemisch aus verschiedenen Epoxidharzen ist.

Die basenkatalysierte Additions-oder Substitutionsreaktion kann mit niedermolekularen Verbindungen (Monomeren), mit Oligomeren, mit polymeren Verbindungen oder mit einem Gemisch dieser Verbindungen durchgeführt werden. Beispiele für Reaktionen, die sowohl an Monomeren als auch an Oligomeren/Polymeren mit den erfindungsgemässen Photoinitiatoren durchgeführt werden können, sind die Knoevenagel-Reaktion oder die Michael-Addition.

Von besonderer Bedeutung sind Zusammensetzungen, worin die Komponente B) ein anionisch polymerisierbares oder vernetzbares organisches Material ist.
Das organische Material kann in Form von einfach- oder mehrfach-funktionellen Monomeren, Oligomeren oder Polymeren vorliegen.

Besonders bevorzugte oligomere/polymere Systeme sind Bindemittel bzw. Lacksysteme wie sie in der Beschichtungsindustrie üblich sind.

Beispiele für solche basisch katalysierbare Bindemittel, bzw. Lacksysteme sind:
a) Acrylatcopolymere mit Alkoxysilan- bzw. Alkoxysiloxan-Seitengruppen, beispielsweise die im US 4772672 oder US 4444974 beschriebenen Polymeren;
b) Zweikomponentensysteme aus hydroxylgruppenhaltigen Polyacrylaten, Polyestern und/oder Polyethern und aliphatischen oder aromatischen Polyisocyanaten;
c) Zweikomponentensysteme aus funktionellen Polyacrylaten und einem Polyepoxid, wobei das Polyacrylat Carboxyl- oder Anhydridgruppen enthält;
d) Zweikomponentensysteme aus fluormodifizierten oder siliconmodifizierten hydroxylgruppenhaltigen Polyacrylaten, Polyestern und/oder Polyethern und aliphatischen oder aromatischen Polyisocyanaten;
e) Zweikomponentensysteme aus (Poly)ketiminen und aliphatischen oder aromatischen Polyisocyanaten;
f) Zweikomponentensysteme aus (Poly)ketiminen und ungesättigten Acrylatharzen oder Acetoacetatharzen oder Methyl-α-acrylamido-methylglykolat;
h) Zweikomponentensysteme aus (Poly)oxazolidinen und anhydridgruppenhaltigen Polyacrylaten oder ungesättigten Acrylatharzen oder Polyisocyanaten;
i) Zweikomponentensysteme aus epoxygruppenhaltigen Polyacrylaten und carboxylgruppenhaltigen Polyacrylaten;
I) Polymere auf Basis von Allyl-glycidylether;
m) Zweikomponentensysteme aus einem (Poly)alkohol und einem (Poly)isocyanat;
n) Zweikomponentensysteme aus einer α,β-ethylenisch ungesättigten Carbonylverbindung und einem aktivierte CH₂-Gruppen enthaltendem Polymer, wobei die aktivierten CH₂-Gruppen entweder in der Hauptkette oder in der Nebenkette oder in beiden enthalten sein können, wie dies beispielsweise in der EP 161697 für (Poly)malonatgruppen beschrieben ist. Andere Verbindungen mit aktivierten CH₂-Gruppen sind (Poly)acetoacetate und (Poly)cyanoacetate.

Innerhalb dieser basisch katalysierbaren Bindemittel sind folgende besonders bevorzugt:
b) Zweikomponentensysteme aus hydroxylgruppenhaltigen Polyacrylaten, Polyestern und/oder Polyethem und aliphatischen oder aromatischen Polyisocyanaten;
c) Zweikomponentensysteme aus funktionellen Polyacrylaten und einem Polyepoxid, wobei das Polyacrylat Carboxyl- oder Anhydridgruppen enthält;
i) Zweikomponentensysteme aus epoxygruppenhaltigen Polyacrylaten und carboxylgruppenhaltigen Polyacrylaten;
m) Zweikomponentensysteme aus einem (Poly)alkohol und einem (Poly)isocyanat, und
n) Zweikomponentensysteme aus einer α,β-ethylenisch ungesättigten Carbonylverbindung und einem aktivierte CH₂-Gruppen enthaltendem Polymer, wobei die aktivierten CH₂-Gruppen entweder in der Hauptkette oder in der Nebenkette oder in beiden enthalten sein können. Andere Verbindungen mit aktivierten CH₂-Gruppen sind (Poly)acetoacetate und (Poly)-cyanoacetate.

Zweikomponentensysteme aus einer α,β-ethylenisch ungesättigten Carbonylverbindung und einem (Poly)malonat sowie deren Herstellung sind in der EP 161687 beschrieben. Die Malonatgruppe kann dabei in einem Polyurethan, Polyester, Polyacrylat, Epoxidharz, Polyamid oder Polyvinyl Polymer entweder in der Hauptkette oder in einer Seitenkette gebunden sein. Als α,β-ethylenisch ungesättigten Carbonylverbindung kann jede durch eine Carbonylgruppe aktivierte Doppelbindung eingesetzt werden. Beispiele sind Ester oder Amide der Acrylsäure oder Methacrylsäure. In den Estergruppen können auch zusätzliche Hydroxylgruppen vorhanden sein. Auch Di- und Triester sind möglich.
Typisch sind zum Beispiel Hexandioldiacrylat oder Trimethylolpropantriacrylat. Anstelle der Acrylsäure können auch andere Säuren und deren Ester oder Amide verwendet werden wie zum Beispiel Crotonsäure oder Zimtsäure.

Die Komponenten des Systems reagieren unter Basenkatalyse bei Raumtemperatur miteinander und bilden ein vernetztes Beschichtungssystem, welches für viele Anwendungen geeignet ist. Aufgrund seiner bereits guten Witterungsbeständigkeit ist es zum Beispiel auch für Aussenanwendungen geeignet und kann bei Bedarf zusätzlich durch UV-Absorber und andere Lichtschutzmittel stabilisiert werden.

Die Zusammensetzungen enthalten den Photoinitiator, Komponente A), vorzugsweise in einer Menge von 0,01 bis 10 Gew.-%, bezogen auf die Komponente B).

Die photopolymerisierbaren Gemische können ausser dem Photoinitiator, Komponente A), verschiedene Additive enthalten. Beispiele hierfür sind thermische Inhibitoren, die eine vorzeitige Polymerisation verhindern sollen, wie z.B. Hydrochinon, Hydrochinonderivate, p-Methoxyphenol, β-Naphthol oder sterisch gehinderte Phenole wie z.B. 2,6-Di(tert-butyl)-pkresol. Zur Erhöhung der Dunkellagerstabilität können z.B. Kupferverbindungen, wie Kupfernaphthenat, -stearat oder -octat, Phosphorverbindungen, wie z.B. Triphenylphosphin, Tributylphosphin, Triethylphosphit, Triphenylphosphit oder Tribenzylphosphit, quartäre Ammoniumverbindungen, wie z.B. Tetramethylammoniumchlorid oder Trimethylbenzylammoniumchlorid, oder Hydroxylaminderivate, wie z.B. N-Diethyl-hydroxylamin verwendet werden. Zwecks Ausschluss des Luftsauerstoffes während der Polymerisation können Paraffin oder ähnliche wachsartige Stoffe zugesetzt werden, die bei Beginn der Polymerisation wegen mangelnder Löslichkeit im Polymeren an die Oberfläche wandern und eine transparente Oberflächenschicht bilden, welche den Zutritt von Luft verhindert. Ebenso kann eine sauerstoffundurchlässige Schicht aufgetragen werden. Als Lichtschutzmittel können in geringer Menge UV-Absorber, wie z.B. solche vom Hydroxyphenyl-benztriazol-, Hydroxyphenyl-benzophenon-, Oxalsäureamid- oder Hydroxyphenyl-s-triazin-Typ, zugesetzt werden. Es können einzelne oder Mischungen dieser Verbindungen mit oder ohne Einsatz von sterisch gehinderten Aminen (HALS) verwendet werden.

Beispiele für solche UV-Absorber und Lichtschutzmittel sind nachfolgend angegeben.
1. 2-(2'-Hydroxyphenyl)-benzotriazole, wie z.B. 2-(2'-Hydroxy-5'-methylphenyl)-1, 2-(3',5'-Ditert-butyl-2'-hydroxyphenyl)-benzotriazol, 2-(5'-tert-Butyl-2'-hydroxyphenyl)-benzo-triazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotrizol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hy-droxy-5'-(2-octyloxycarbonylethyl)phenyl)-5-chlor-benzotriazol, 2-(3'-tert-Butyl-5'-[2-(2-ethyl-hexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbo-nylethyl)-phenyl)-benzotriazol, 2-(3'-tert-Butyl-5'-[2-(2-ethylhexyloxy)carbonylethyl]-2'-hydro-xyphenyl)-benzotriazol, 2-(3'-Dodecyl-2'-hydroxy-5'-methylphenyl)-benzotriazol, und 2-(3'-tart-Butyl-2'hydroxy- 5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; [R-CH₂₋ CH₂-COO(CH₂)₃]₂- mit R = 3'-tert-Butyl-4'-hydroxy-5'-2H-benzotriazol-2-yl-phenyl.
2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
3. Ester von gegebenenfalls substituierten Benzoesäuren, wie z.B. 4-tert-Butyl-phenylsali-cylat, Phenylsalicylat, Octylphenyl-salicylat, Dibenzoylresorcin, Bis-(4-tert-butylbenzoyl)-re-sorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert- butyl-4-hydroxybenzoesäure-octadecylester, 3,5-Di-tert-butyl-4-hydroxybenzoesäure-2-methyl-4,6-di-tert-butylphenylester.
4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
5. Sterisch gehinderte Amine, wie z.B. Bis-(2,2,6,6-tetramethyl-piperidyl)-sebacat, Bis-(2,2,-6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-plperidyl)-hexa-methylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarboxylat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-nbutyl-2-(2-hydroxy-3,5-di-tert-butylbenzyl)-malonat, 3-n-Octyl-7,7,9,9-tetramethyl-1,3,8-triazaspiro-[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetra-methyl-4-piperidyl)-hexamethylendiamin und 4-Morpholino-2,6-dichlor-1,3,5-triazin, Konden-sationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Do-decyl-1-(2,2,6,6-tetramethyl-4-piperidyl)pyrrolidin-2,5-dion, 3-Dodecyl-1-(1,2,2,6,6-penta-methyl-4-piperidyl)-pyrrolidin-2,5-dion.
6. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyloxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
7. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxy-phenyl)-6-(2,4-dimethylphenyl)-1,3,5-triazin, 2-(2-Hydroxy-4-octyloxyphenyl)-4,6-bis(4-me-thylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethyl-phenyl)-1,3,5-triazin, 2-[2-hydroxy-4-(2-hydroxy-3-octyloxy-propyloxy)phenyl]-4,6-bis(2,4-dimethylphenyl)-1,3,5-triazin, 2-[4-dodecyl/tridecyl-oxy-(2-hydroxypropyl)oxy-2-hydroxy-phenyl]-4,6-bis(2,4-dimethylphenyl)-1,3,5-triazin.
8. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpenta-erythritdiphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tertbutyl-6-methylphenyl)-pentaerythritdiphosphit, Bis-(2,4,6-tri-tert-butylphenyl)-penta-erythritdiphosphit, Tristearyl-sorbit-triphosphit, Tetrakis-(2,4-di-tert-butylphenyl)-4,4'-biphen-ylendiphosphonit, 6-lsooctyloxy-2,4,8,10-tetra-tert-butyl-12H-dibenz[d,g]-1,3,2-dioxaphos-phocin, 6-Fluor-2,4,8,10-tetra-tert-butyl-12-methyl-dibenz[d,g]-1,3,2-dioxaphosphocin, Bis-(2,4-ditert-butyl-6-methylphenyl)-methylphosphit, Bis-(2,4-di-tert-butyl-6-methylphenyl)-ethylphosphit.

### Beispiele für weitere Zusätze sind:

Füllstoffe und Verstärkungsmittel, wie z.B. Calciumcarbonat, Silikate, Glasfasern, Glaskugeln, Asbest, Talk, Kaolin, Glimmer, Bariumsulfat, Metalloxide und -hydroxide, Ruß, Graphit, Holzmehl und Mehle oder Fasern anderer Naturprodukte, synthetische Fasem.
Sonstige Zusätze, wie z.B. Weichmacher, Gleitmittel, Emulgatoren, Pigmente, Rheologieadditive, Katalysatoren, Verlaufshilfsmittel, optische Aufheller, Flammschutzmittel, Antistatika, Treibmittel.

Neben den vorstehend angegebenen Additiven können noch zusätzliche Coinitiatoren vorhanden sein. In der Regel handelt es sich dabei um Farbstoffe, die beispielsweise durch Energieübertragung oder Elektronenübertragung die Gesamtquantenausbeute verbessern. Geeignete Farbstoffe, welche als Coinitiatoren zugesetzt werden können, sind z.B. Triarylmethane, z.B. Malachit Grün, Indoline, Thiazine, z.B. Methylen Blau, Xanthone, Thioxanthone, Oxazine, Acridine oder Phenazine, z.B. Safranin, und Rhodamine der Formel 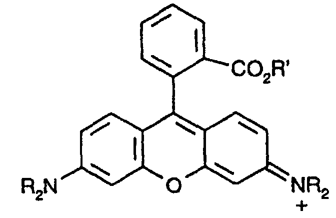 worin R für Alkyl- oder Arylreste steht und R' Wasserstoff, einen Alkyl- oder Arylrest darstellt, z.B. Rhodamin B, Rhodamin 6G oder Violamin R, ausserdem Sulforhodamin B oder Sulforhodamin G.

Bevorzugt sind Thioxanthone, Oxazine, Acridine, Phenazine und Rhodamine.
Ebenfalls geeignet sind in diesem Zusammenhang Kombinationen von Farbstoffen mit Boraten wie sie beispielsweise in US 4772530, GB 2307474, GB 2307473, GB 2307472 und EP 775706 beschrieben sind.

Neben den vorstehend beschriebenen basisch katalysierbaren (härtbaren) Bindemitteln, Komponente B), kann die Zusammensetzung auch noch weitere Bindemittel enthalten. Möglich sind zum Beispiel weitere olefinisch ungesättigte Verbindungen. Die ungesättigten Verbindungen können eine oder mehrere olefinische Doppelbindungen enthalten. Sie können niedermolekular (monomer) oder höhermolekular (oligomer) sein. Beispiele für Monomere mit einer Doppelbindung sind Alkyl- oder Hydroxyalkylacrylate oder -methacrylate, wie z.B. Methyl-, Ethyl-, Butyl-, 2-Ethylhexyl- oder 2-Hydroxyethylacrylat, Isobornylacrylat, Methyl- oder Ethylmethacrylat. Interessant sind auch Siliconacrylate. Weitere Beispiele sind Acrylnitril, Acrylamid, Methacrylamid, N-substituierte (Meth)acrylamide, Vinylester wie Vinylacetat, Vinylether wie Isobutylvinylether, Styrol, Alkyl- und Halogenstyrole, N-Vinylpyrrolidon, Vinylchlorid oder Vinylidenchlorid.

Beispiele für Monomere mit mehreren Doppelbindungen sind Ethylenglykol-, Propylenglykol-, Neopentylglykol-, Hexamethylenglykol- oder Bisphenol-A-diacrylat, 4,4'-Bis(2-acryloyloxyethoxy)-diphenylpropan, Trimethylolpropan-triacrylat, Pentaerythrittriacrylat oder -tetraacrylat, Vinylacrylat, Divinylbenzol, Divinylsuccinat, Diallylphthalat, Triallylphosphat, Triallylisocyanurat oder Tris-(2-acryloylethyl)isocyanurat.

Beispiele für höhermolekulare (oligomere) mehrfach ungesättigte Verbindungen sind acrylierte Epoxidharze, acrylierte oder Vinylether- oder Epoxy-Gruppen enthaltende Polyester, Polyurethane und Polyether. Weitere Beispiele für ungesättigte Oligomere sind ungesättigte Polyesterharze, die meist aus Maleinsäure, Phthalsäure und einem oder mehreren Diolen hergestellt werden und Molekulargewichte von etwa 500 bis 3000 besitzen. Daneben können auch Vinylether-Monomere und -Oligomere, sowie maleat-terminierte Oligomere mit Polyester-, Polyurethan-, Polyether-, Polyvinylether- und Epoxidhauptketten eingesetzt werden. Insbesondere Kombinationen von Vinylethergruppen tragenden Oligomeren und Polymeren, wie sie in der WO 90/01512 beschrieben sind, sind gut geeignet. Aber auch Copolymere aus Vinylether und Maleinsäure funktionalisierten Monomeren kommen in Frage. Solche ungesättigten Oligomere kann man auch als Prepolymere bezeichnen.

Besonders geeignet sind z.B. Ester von ethylenisch ungesättigten Carbonsäuren und Polyolen oder Polyepoxiden, und Polymere mit ethylenisch ungesättigten Gruppen in der Kette oder in Seitengruppen, wie z. B. ungesättigte Polyester, Polyamide und Polyurethane und Copolymere hiervon, Alkydharze, Polybutadien und Butadien-Copolymere, Polyisopren und Isopren-Copolymere, Polymere und Coplymere mit (Meth)Acrylgruppen in Seitenketten, sowie Mischungen von einem oder mehreren solcher Polymerer.

Werden solche radikalisch härtbaren Monomeren, Oligomeren/Polymeren zusätzlich verwendet, so ist es zweckmässig, aber nicht unbedingt erforderlich, einen weiteren radikalisch zerfallenden Photoinitiator zuzusetzen. Solche Photoinitiatoren sind bekannt und werden grosstechnisch hergestellt. Beispiele sind Benzophenon, Benzophenonderivate, Acetophenon, Acetophenonderivate, wie beispielsweise α-Hydroxycycloalkylphenylketone, Dialkoxyacetophenone, α-Hydroxy- oder α-Aminoacetophenone, 4-Aroyl-1,3-Dioxolane, Benzoinalkytether und Benzilketale, Monoacylphosphinoxide, Bisacylphosphinoxide, Ferrocene oder Titanocene.
Beispiele sind in der EP 284561 genannt. Derartige Polymersysteme, bei denen die Härtung/Vernetzung nach unterschiedlichen Mechanismen erfolgt, werden auch als Hybridsysteme bezeichnet.

Den erfindungsgemässen Zusammensetzungen können auch nicht reaktive Bindemittel zugesetzt werden, was besonders zweckmässig ist, wenn es sich bei den photopolymerisierbaren Verbindungen um flüssige oder viskose Substanzen handelt. Die Menge des nicht reaktiven Bindemittels kann z.B. 5-95, vorzugsweise 10-90 und besonders 40-90 Gew.-% betragen, bezogen auf den Gesamtfestkörper. Die Wahl des nicht reaktiven Bindemittels erfolgt je nach dem Anwendungsgebiet und den hierfür geforderten Eigenschaften wie zum Beispiel Entwickelbarkeit in wässrigen und organischen Lösungsmittelsystemen, Adhäsion auf Substraten und Sauerstoffempfindlichkeit.

Geeignete Bindemittel sind z.B. Polymere mit einem Molekulargewicht von etwa 5000-2000000, bevorzugt 10000-1000000. Beispiele sind: Homo- und Copolymere Acrylate und Methacrylate, z.B. Copolymere aus Methylmethacrylat/Ethylacrylat/Methacrylsäure, Poly-(methacrylsäurealkylester), Poly(acrylsäurealkylester); Celluloseester und -ether wie Celluloseacetat, Celluloseacetatbutyrat, Methylcellulose, Ethylcellulose; Polyvinylbutyral, Polyvinylformal, cyclisierter Kautschuk, Polyether wie Polyethylenoxid, Polypropylenoxid, Polytetrahydrofuran; Polystyrol, Polycarbonat, Polyurethan, chlorierte Polyolefine, Polyvinylchlorid, Copolymere aus Vinylchlorid/Vinylidenchlorid, Copolymere von Vinylidenchlorid mit Acrylnitril, Methylmethacrylat und Vinylacetat, Polyvinylacetat, Copoly(ethylen/vinylacetat), Polymere wie Polycaprolactam und Poly(hexamethylenadipamid), Polyester wie Poly(ethylenglykolterephthalat) und Poly(hexamethylenglykolsuccinat).

Ebenfalls Gegenstand der Erfindung ist ein Verfahren zur Durchführung basenkatalysierter Reaktionen, welches dadurch gekennzeichnet ist, dass eine erfindungsgemässe Zusammensetzung wie vorstehend beschrieben, mit Licht einer Wellenlänge von 200 nm bis 650 nm bestrahlt wird.

In manchen Fällen kann es vorteilhaft sein, dass während oder nach der Belichtung erwärmt wird. Auf diese Weise lässt sich die Vernetzungsreaktion vielfach noch beschleunigen.

Die Lichtempfindlichkeit der erfindungsgemässen Zusammensetzungen reicht in der Regel von ca. 200 nm über das UV-Gebiet bis in den Infrarotbereich (ca. 20000 nm, insbesondere 1200 nm) und umspannt somit einen sehr breiten Bereich. Geeignete Strahlung enthält z.B. Sonnenlicht oder Licht, aus künstlichen Lichtquellen. Als Lichtquellen kommen daher eine grosse Anzahl der verschiedensten Typen zur Anwendung. Es sind sowohl Punktquellen als auch flächenförmige Strahler (Lampenteppiche) geeignet. Beispiele sind: Kohlelichtbogenlampen, Xenon-Lichtbogenlampen, Quecksilbermitteldruck-, -hochdruck- und -niederdruckstrahler, gegebenenfalls mit Metall-Halogeniden dotiert (Metall-Halogenlampen), mikrowellenangeregte Metalldampflampen, Excimer Lampen, superaktinische Leuchtstoffröhren, Fluoreszenzlampen, Argonglühlampen, Elektronenblitzlampen, photographische Flutlichtlampen, Elektronenstrahlen und Röntgenstrahlen, erzeugt mittels Synchrotronen oder Laser-Plasma. Der Abstand zwischen Lampe und erfindungsgemässem zu belichtenden Substrat kann je nach Anwendungszweck und Lampentyp bzw. -stärke variieren, z.B. zwischen 2 cm bis 150 cm. Speziell geeignet sind auch Laserlichtquellen, z.B. Excimer-Laser. Auch Laser im sichtbaren Bereich oder im IR-Bereich können eingesetzt werden. Hier ist die hohe Empfindlichkeit der erfindungsgemässen Materialien und die Möglichkeit der Anpassung eines Farbstoffes als Coinitiator an die Laserlinie sehr vorteilhaft. Nach dieser Methode können gedruckte Schaltungen in der Elektronikindustrie, lithographische Offsetdruckplatten oder Reliefdruckplatten sowie photographische Bildaufzeichnungsmaterialien hergestellt werden.

Die erfindungsgemässen Zusammensetzungen können für verschiedene Zwecke eingesetzt werden, beispielsweise als Druckfarbe, als Klarlack, als Weisslack, z.B. für Holz oder Metall, als Anstrichstoff, u.a. für Papier, Holz, Metall oder Kunststoff, als Pulverlack, als tageslichthärtbarer Anstrich für Bauten- und Straßenmarkierung, für photographische Reproduktionsverfahren, für holographische Aufzeichnungsmaterialien, für Bildaufzeichnungsverfahren oder zur Herstellung von Druckplatten, die mit organischen Lösemitteln oder wässrigalkalisch entwickelbar sind, zur Herstellung von Masken für den Siebdruck, als Zahnfüllmassen, als Klebstoffe, als drucksensitive Klebstoffe, als Laminierharze, als Aetz- oder Permanentresists und als Lötstoppmasken für elektronische Schaltungen, zur Herstellung von dreidimensionalen Gegenständen durch Massenhärtung (UV-Härtung in transparenten Formen) oder nach dem Stereolithographieverfahren, wie es z.B. in US 4575330 beschrieben ist, zur Herstellung von Verbundwerkstoffen (z.B. styrolischen Polyestern, die gegebenenfalls Glasfasern und/oder andere Fasern und andere Hilfsstoffe, enthalten können) und anderen dickschichtigen Massen, zur Beschichtung oder Versiegelung von elektronischen Teilen oder als Überzüge für optische Fasern.

In Lacken verwendet man häufig Gemische eines Prepolymeren mit mehrfach ungesättigten Monomeren, die ausserdem noch ein einfach ungesättigtes Monomer enthalten. Das Prepolymere ist hierbei in erster Linie für die Eigenschaften des Lackfilmes massgebend, durch seine Variation kann der Fachmann die Eigenschaften des gehärteten Filmes beeinflussen. Das mehrfach ungesättigte Monomere fungiert als Vernetzer, das den Lackfilm unlöslich macht. Das einfach ungesättigte Monomere fungiert als reaktiver Verdünner, mit dessen Hilfe die Viskosität herabgesetzt wird, ohne dass ein Lösungsmittel verwendet werden muss.

Ungesättigte Polyesterharze werden meist in Zweikomponentensystemen zusammen mit einem einfach ungesättigten Monomer, vorzugsweise mit Styrol, verwendet. Für Photoresists werden oft spezifische Einkomponentensysteme verwendet, wie z.B. Polymaleinimide, Polychalkone oder Polyimide, wie sie in der DE 2308830 beschrieben sind.
Die erfindungsgemässen photohärtbaren Zusammensetzungen eignen sich z.B. als Beschichtungsstoffe für Substrate aller Art, z.B. Holz, Textilien, Papier, Keramik, Glas, Kunststoffe wie Polyester, Polyethylenterephthalat, Polyolefine oder Celluloseacetat, insbesondere in Form von Filmen, sowie Metalle wie Al, Cu, Ni, Fe, Zn, Mg oder Co und GaAs, Si oder SiO₂, auf denen eine Schutzschicht oder durch bildmässiges Belichten eine Abbildung aufgebracht werden soll.

Die Beschichtung der Substrate kann erfolgen, indem eine flüssige Zusammensetzung, eine Lösung oder Suspension auf das Substrat aufgebracht wird. Die Wahl des Lösungsmittels und die Konzentration richten sich hauptsächlich nach der Art der Zusammensetzung und nach dem Beschichtungsverfahren. Das Lösungsmittel soll inert sein, d.h. es soll mit den Komponenten keine chemische Reaktion eingehen und es soll bei der Trocknung nach dem Beschichten wieder entfernt werden können. Geeignete Lösungsmittel sind z.B. Ketone, Ether und Ester, wie Methylethylketon, Isobutylmethylketon, Cyclopentanon, Cyclohexanon, N-Methylpyrrolidon, Dioxan, Tetrahydrofuran, 2-Methoxyethanol, 2-Ethoxyethanol, 1-Methoxy-2-propanol, 1,2-Dimethoxyethan, Essigsäureethylester, Essigsäure-n-butylester und 3-Ethoxy-propionsäureethylester.
Die Lösung wird mittels bekannter Beschichtungsverfahren gleichförmig auf ein Substrat aufgebracht, z.B. durch Schleudern, Tauchen, Rakelbeschichtung, Vorhanggiessverfahren, Aufpinseln, Sprühen, speziell durch elektrostatisches Sprühen und Reverse-Roll-Beschichtung, sowie durch elektrophoretische Abscheidung. Es ist auch möglich, die lichtempfindliche Schicht auf einen temporären, flexiblen Träger zu bringen und dann durch Schichtübertragung via Lamination das endgültige Substrat, z.B. eine kupferkaschierte Leiterplatte, zu beschichten.

Die Auftragsmenge (Schichtdicke) und Art des Substrates (Schichtträger) sind abhängig vom gewünschten Applikationsgebiet. Der Schichtdickenbereich umfasst im allgemeinen Werte von ca. 0,1 µm bis mehr als 100 µm.

Die erfindungsgemässen strahlungsempfindlichen Zusammensetzungen können auch bildmässig belichtet werden. Sie finden dann Anwendung als Negativresists. Sie eignen sich für die Elektronik (Galvanoresist, Aetzresist, Lötstopresist), die Herstellung von Druckplatten, wie Offsetdruckplatten, Flexo- und Hochdruckplatten oder Siebdruckformen, die Herstellung von Stempeln, den Einsatz beim Formteilätzen oder den Einsatz als Mikroresist bei der Herstellung integrierter Schaltkreise. Dementsprechend unterschiedlich sind die möglichen Schichträger und die Verarbeitungsbedingungen der beschichteten Substrate.

Der Begriff "bildmässige" Belichtung beinhaltet sowohl die Belichtung durch eine Photomaske, die ein vorbestimmtes Muster enthält, beispielsweise ein Diapositiv, die Belichtung durch einen Laserstrahl, der beispielsweise computergesteuert über die Oberfläche des beschichteteten Substrates bewegt wird und auf diese Weise ein Bild erzeugt, sowie die Bestrahlung mit computergesteuerten Elektronenstrahlen.
Nach der bildmässigen Belichtung des Materials und vor der Entwicklung kann es vorteilhaft sein, für kürzere Zeit ein thermische Behandlung durchzuführen. Dabei werden nur die belichteten Teile thermisch gehärtet. Die angewandten Temperaturen liegen im allgemeinen bei 50-150°C, bevorzugt bei 80-130°C; die Zeit für die thermische Behandlung liegt in der Regel zwischen 0,25 und 10 Minuten.

Ein weiteres Einsatzgebiet der Photohärtung ist die Metallbeschichtung, beispielsweise bei der Lackierung von Blechen und Tuben, Dosen oder Flaschenverschlüssen, sowie die Photohärtung auf Kunststoffbeschichtungen, beispielsweise von Fussboden- oder Wand-belägen auf PVC-Basis.
Beispiele für die Photohärtung von Papierbeschichtungen sind die farblose Lackierung von Etiketten, Schallplattenhüllen oder Buchumschlägen.

Ein weiterer Gegenstand der Erfindung ist die Verwendung einer Verbindung der Formel I, wie vorstehend beschrieben, als Photoinitiator für photochemisch induzierte basenkatalysierte Additions- oder Substitutionsreaktionen, insbesondere zur Härtung von Formteilen aus Verbundmassen, worin die Reste R₁, R₂, R₃, R₄, R₅, R₁₂, R₁₃, R₁₄, R₁₅ und m die vorstehend angegebenen Bedeutungen und Bevorzugungen haben.

Ebenfalls Gegenstand der Erfindung ist die vorstehend genannte Verwendung zur Herstellung von Beschichtungen, Formmassen oder photostrukturierten Schichten.

Die Verbundmasse besteht in der Regel aus einem selbsttragenden Matrixmaterial, z.B. einem Glasfasergewebe, oder auch beispielsweise Pflanzenfasern [vgl. K.-P. Mieck, T. Reussmann in Kunststoffe 85 (1995), 366-370], das mit der lichthärtenden Formulierung durchtränkt wird. Mit den erfindungsgemässen Verbindungen hergestellte Formteile aus Verbundmassen erreichen eine hohe mechanische Stabilität und Widerstandsfähigkeit. Die erfindungsgemässen Verbindungen sind auch als Photohärter in Form-, Tränk- und Überzugsmassen, wie sie beispielsweise in der EP 7086 beschrieben sind, einsetzbar. Solche Massen sind beispielsweise Feinschichtharze, an die hohe Anforderungen bezüglich der Härtungsaktivität und Vergilbungsresistenz gestellt werden, faserverstärkte Formstoffe, wie z.B. plane, längs- oder quergewellte Lichtplatten.

Beispiele und Bevorzugungen für basenkatalysierte Additions- oder Substitutionsreaktionen sind vorstehend genannt.
Ein weiterer Gegenstand der Erfindung ist ein beschichtetes Substrat, das auf mindestens einer Oberfläche mit einer wie oben beschriebenen Zusammensetzung beschichtet ist, sowie ein Verfahren zur photographischen Herstellung von Reliefabbildungen, in welchem ein beschichtetes Substrat bildmässig belichtet wird und danach die unbelichteten Anteile mit einem Lösemittel entfernt werden. Insbesondere interessant ist dabei die vorstehend erwähnte Belichtung mittels eines Laserstrahls.
Gegenstand der Erfindung ist auch die Verwendung der erfindungsgemässen Verbindungen zur Herstellung von Beschichtungen, Formmassen oder photostrukturierten Schichten.
Ebenfalls Gegenstand der Erfindung sind polymerisierte oder vernetzte erfindungsgemässe Zusammensetzungen.

Die nachfolgenden Beispiele erläutern die Erfindung weiter. Angaben in Teilen oder Prozenten beziehen sich, ebenso wie in der übrigen Beschreibung und in den Patentansprüchen, auf das Gewicht, sofern nichts anderes angegeben ist. Wenn Alkyl- oder Alkoxyreste mit mehr als drei C-Atomen ohne Hinweis auf deren isomere Form angegeben sind, so beziehen sich die Angaben auf die jeweiligen n-lsomere.
In den Beispielen werden folgende Abkürzungen verwendet:
"Ar" für Aryl, "DMSO" für Dimethylsulfoxid, "I.R." für Infrarot Spektren, "¹H NMR" für Wasserstoff-Kernresonanzspektren (Angabe der Verschiebungswerte in ppm), "m.p." für Schmelzpunkt.

### Beispiele A: Herstellung der Bromide

### Allgemeines Verfahren:

Ein Äquivalent der jeweiligen Stickstoffbase wird in Toluol bei Raumtemperatur gerührt. Ein Äquivalent 3-Bromo-2-phenylpropen in Toluol wird zugegeben und das Reaktionsgemisch über Nacht gerührt. Das ausgefallene Bromid wird filtriert und mit Toluol gewaschen. Man erhält das Produkt in Ausbeuten von 60 bis 70 %.

### Beispiel A1

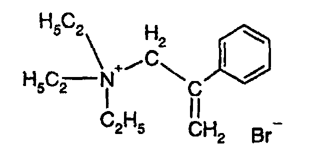

Analyse berechnet für C₁₅H₂₄BrN • 1 H₂O: C 56.97; H 8.29; N 4.43. Gefunden: C 56.92; H 8.30; N 4.19.
m.p.: 90-93°C.
I.R. (KBr) 1617 cm⁻¹.
¹H NMR (d₆-DMSO): 7.56 (2H, m, ArH), 7.40 (3H, m, ArH), 5.80 (1H, s, =CH), 5.74 (1H, s,
=CH), 4.41 (2H, s, N⁺-CH₂), 3.07 (6H, q, J = 7.2Hz), 1.11 (9H, t, J = 7.1Hz).
m/z (ESI-MS) : 218 (Ammonium Kation); 79 und 81 (Bromid Anion).

### Beispiel A2

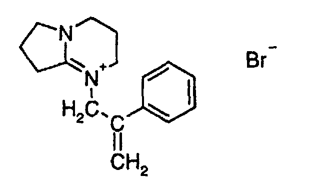

¹H NMR (d₆-DMSO): 7.52-7.10 (5H, m, ArH), 5.56 (1H, s, =CH), 5.24 (1H, s, =CH), 4.56 (2H, s, N⁺-CH₂), 3.68 (2H, t, J = 7.2Hz), 3.38 (4H, m), 3.01 (2H, d, J = 7.8Hz), 2.05 (4H, m). m/z : (ESI-TOF-MS): 241 (Ammonium Kation).

### Beispiele B: Herstellung der Boratsalze

### Allgemeines Verfahren zur Herstellung der Tetraphenylborat-Salze

Ein Äquivalent des jeweiligen Bromids wird bei Raumtemperatur in Wasser gerührt und ein Äquivalent einer wässrigen Lösung von Natriumtetraphenylborat wird zugegeben. Das ausgefallene Tetraphenylboratsalz wird abfiltriert, mit Wasser gewaschen und unter Vakuum getrocknet. Man erhält das Produkt in einer Ausbeute von 95%.

### Beispiel B1

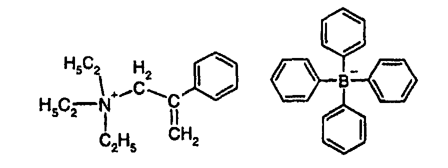

Analyse berechnet für C₃₉H₄₄BN 2 H₂O : C 81.66; H 8.43; N 2.44. Gefunden: C 81.31; H 7.94; N 2.24.
m.p. = 165-166°C. I.R. (KBr) 1579 cm⁻¹.
¹H NMR (d₆-DMSO): 7.55 (2H, m, ArH), 7.41 (3H, m, ArH), 7.17 (8H, m, ArH), 6.91 (12H, m, ArH), 5.79 (1H, s, =CH), 5.75 (1H, s, =CH), 4.38 (2H, s, N⁺-CH₂), 3.07 (6H, q, J = 7.2Hz), 1.10 (9H, t, J = 7.1Hz).
m/z (ESI-TOF-MS): 218 (Ammonium Kation); 319 (Borat Anion).

### Beispiel B2

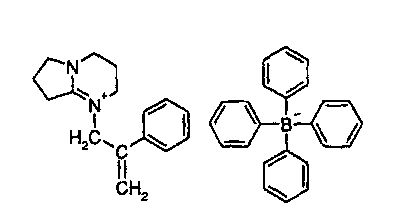

Analyse berechnet für C₄₀H₄₁BN₂ · 0.4 H₂O : C 84.61; H 7.42; N 4.93. Gefunden: C 84.67; H 7.44; N 4.76.
m.p. = 141-142°C.
I.R. (KBr) 1673 cm⁻¹.
¹H NMR (CDCl₃): 7.33 (11H, m, ArH), 7.10 (2H, m, ArH), 6.91 (12H, m, ArH), 5.37 (1H, s, =CH), 4.87 (1H, s, =CH), 3.61 (2H, s, N⁺-CH₂), 2.92 (2H, t, J = 7.2Hz), 2.66 (2H, t, J = 5.5Hz), 2.48 (2H, t, J = 5.8Hz), 1.93 (2H, t, J = 7.6Hz), 1.51 (2H, m), 1.36 (2H, t, J = 5.7Hz). m/z (ESI-MS) : 241 (Ammonium Kation); 319 (Borat Anion).

### Beispiel B3

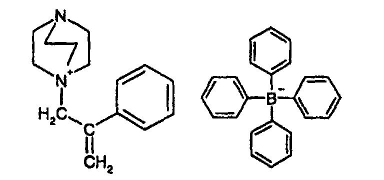

Analyse berechnet für C₃₉H₄₁BN₂ 0.75 H₂O : C 83.34; H 7.62; N 4.98. Gefunden: C 83.36; H 7.49; N 4.66.
m.p. = 222-223°C.
¹H NMR (d₆-DMSO): 7.58 (2H, m, ArH), 7.42 (3H, m, ArH), 7.17 (8H, m, ArH), 6.92(8H, m, ArH), 6.78 (4H, m, ArH), 5.93 (1H, s, =CH), 5.71 (1H, s, =CH), 4.43 (2H, s, N⁺-CH₂), 3.14 (6H, m), 2.93 (6H, m).
m/z (ESI-TOF-MS): 229 (Ammonium Kation); 319 (Borat Anion).

### Allgemeines Verfahren zur Herstellung der Tris(3-fluorophenyl)hexylborat-Salzes:

Ein Äquivalent des jeweiligen Bromids wird bei Raumtemperatur in Wasser gerührt und ein Äquivalent einer methanolischen Lösung von Tetramethylammonium-tris(3-fluorophenyl)hexylborat zugegeben. Das ausgefallene Tris(3-fluorophenyl)hexylboratsalz wird abfiltriert, mit Wasser gewaschen und unter Vakuum getrocknet. Man erhält das Produkt in einer Ausbeute von 85%.

### Beispiel B4

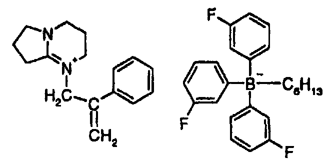

¹H NMR (d₆-DMSO): 7.43 (5H, m, ArH), 6.96 (9H, m, ArH), 6.53 (3H, m, ArH), 5.56 (1H, s, =CH), 5.21 (1H, s, =CH), 4.54 (2H, s, N⁺-CH₂), 3.67 (2H, t, J = 7.2Hz), 3.34 (4H, m), 2.98 (2H, t, J = 7.8Hz), 1.97 (4H, m), 1.57 (6H, br. s.), 0.8 (7H, m).
m/z (ESI-TOF-MS): 241 (Ammonium Kation); 381 (Borat Anion).

### Beispiel B5

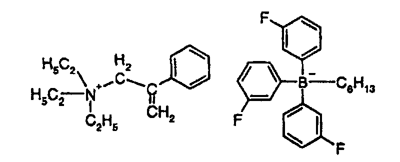

Analyse berechnet für C₃₉H₄₉BF₃N : C 78.12; H 8.24; F 9.50; N 2.33. Gefunden: C 77.90; H 8.32; F 9.41; N 2.80.
m.p. = 72-74°C.
I.R. (KBr) 1594 cm⁻¹.
¹H NMR (d₆-DMSO): 7.55 (2H, m, ArH), 7.41 (3H, m, ArH), 6.95 (6H, m, ArH), 6.78 (3H, m, ArH), 6.55 (3H, m, ArH), 5.79 (1H, s, =CH), 5.75 (1H, s, =CH), 4.38 (2H, s, N⁺-CH₂), 3.07 (6H, q, J = 7.2Hz), 1.12 (15H, m), 0.80 (7H, m).
m/z (ESI-TOF-MS) : 218 (Ammonium Kation); 381 (Borat Anion).

### Applikationsbeispiel C

### Beispiel C1:

Es wird eine Zusammensetzung durch Mischen der folgenden Komponenten hergestellt:

| | |
|---|---|
| 100.0 Teile | Epoxyphenol Novolak (^{RTM}GY1180; Ciba Spezialitätenchemie) |
| 200.0 Teile | Polyacrylat mit Carbonylgruppen (^{RTM}CARBOSET 525; B.F. Goodrich) |
| 9.0 Teile | latente Base (=3%) |
| 1.5 Teile | Isopropylthioxanthon (ITX) (=0.5%) |
| 500.0 Teile | Aceton |

Die so erhaltene Formulierung wird mit einer 100 µm Spiralrakel auf eine Aluminiumblech aufgebracht und 15 Minuten bei 50°C vorgetrocknet. Auf diese Schicht wird eine Polyesterfolie aufgebracht und darauf ein standardisiertes Testnegativ mit 21 Stufen unterschiedlicher optischer Dichte (Stouffer Keil) aufgelegt. Die Probe wird mit einer zweiten UV-transparenten Folie abgedeckt und auf einer Metallplatte mittels Vakuum angepresst. Die Belichtung erfolgt in einer ersten Testreihe während 80 Sekunden, in einer zweiten während 160 Sekunden und in einer dritten während 320 Sekunden im Abstand von 60 cm mittels einer 3kW Metallhalogenid Lampe (ORC SMX3000). Nach der Belichtung werden die Folien und die Maske entfernt und die belichtete Schicht wird in einem Ultraschallbad bei 23°C in Ethanol 5 Minuten lang entwickelt. Die Trocknung erfolgt bei 120°C während 5 Minuten in einem Umluftofen. Die Empfindlichkeit des verwendeten Initiatorsystems wird durch die Angabe der letzten klebefrei abgebildeten Keilstufe charakterisiert. Je höher die Zahl der Stufen ist, desto empfindlicher ist das getestete System.
Die Ergebnisse sind der Tabelle 1 zu entnehmen.

**Tabelle 1**

| Photoinitiator aus Beispiel | Zahl der abgebildeten Stufen nach | | |
|---|---|---|---|
| | 80 Sek. | 160 Sek. | 320 Sek. |
| B1 | 11 | 13 | 15 |
| B2 | 9 | 11 | 13 |

## Patentansprüche

1. Verbindungen der Formel (I) 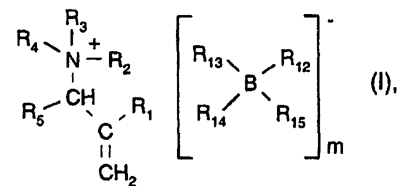 worin
**m** 1 oder 2 ist und der Anzahl positiver Ladungen des Kations entspricht;
**R**_{**1**} Phenyl, Naphthyl, Phenanthryl, Anthracyl, Pyrenyl, 5,6,7,8-Tetrahydro-2-naphthyl, 5,6,7,8-Tetrahydro-1-naphthyl, Thienyl, Benzo[b]thienyl, Naphto[2,3-b]thienyl, Thianthrenyl, Dibenzofuryl, Chromenyl, Xanthenyl, Thioxanthyl, Phenoxathiinyl, Pyrrolyl, Imidazolyl, Pyrazolyl, Pyrazinyl, Pyrimidinyl, Pyridazinyl, Indolizinyl, Isoindolyl, Indolyl, Indazolyl, Purinyl, Chinolizinyl, Isochinolyl, Chinolyl, Phthalazinyl, Naphthyridinyl, Chinoxalinyl, Chinazolinyl, Cinnolinyl, Pteridinyl, Carbazolyl, β-Carbolinyl, Phenanthridinyl, Acridinyl, Perimidinyl, Phenanthrolinyl, Phenazinyl, Isothiazolyl, Phenothiazinyl, Isoxazolyl, Furazanyl, Terphenyl, Stilbenyl, Fluorenyl oder Phenoxazinyl darstellt, wobei diese Reste unsubstituiert oder mit C₁-C₁₈-Alkyl, C₃-C₁₈-Alkenyl, C₃-C₁₈-Alkinyl, C₁-C₁₈-Halogenalkyl, NO₂, NR₆R₇, N₃, OH, CN, OR₈, SR₈, C(O)R₉, C(O)OR₁₀ oder Halogen ein- oder mehrfach substituiert sind, oder R₁ einen Rest der Formeln A oder B 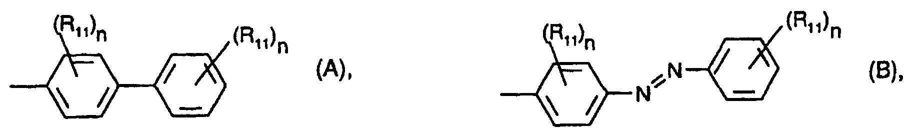 bedeutet;
**R**_{**2**}**, R**_{**3**} und **R**_{**4**} unabhängig voneinander Wasserstoff, C₁-C₁₈-Alkyl, C₃-C₁₈-Alkenyl, C₃-C₁₈-Alkinyl oder Phenyl sind oder unabhängig voneinander R₂ und R₃ und/oder R₄ und R₃ eine C₂-C₁₂-Alkylenbrücke bilden; oder R₂, R₃, R₄ mit dem Stickstoffatom, an welches sie gebunden sind, eine Phosphazen-Base vom Typ P₁, P₂, P₄ oder eine Gruppe der Strukturformeln (a), (b), (c), (d), (e), (f) oder (g) bilden 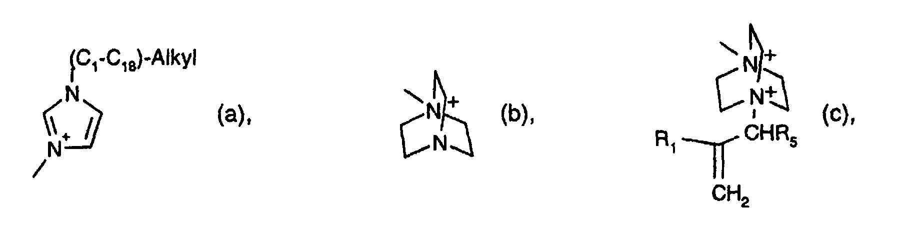 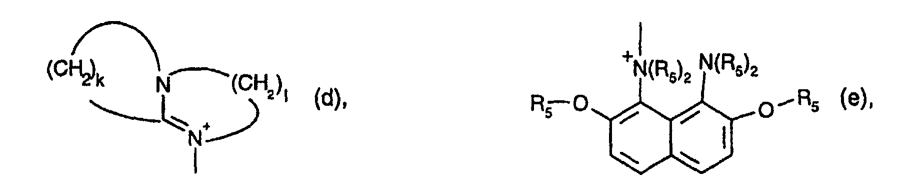 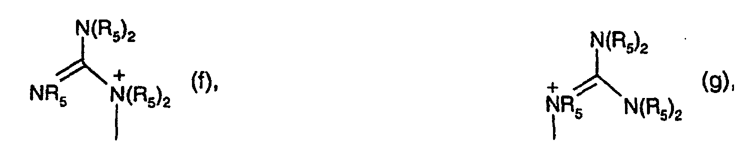 worin
**k** und **l** unabhängig voneinander eine Zahl von 2 bis 12 bedeuten;
**R**_{**5**}**, R**_{**6**}**, R**_{**7**}**, R**_{**8**}**, R**_{**9**} und **R**_{**10**} unabhängig voneinander Wasserstoff oder C₁-C₁₈-Alkyl sind;
**R**_{**11**} C₁-C₁₈-Alkyl, C₂-C₁₈-Alkenyl, C₂-C₁₈-Alkinyl, C₁-C₁₈-Halogenalkyl, NO₂, NR₆R₇, OH, CN, OR₈, SR₈, C(O)R₉, C(O)OR₁₀ oder Halogen ist; und
**n** 0 oder eine Zahl 1, 2 oder 3 ist;
**R**_{**12**}**, R**_{**13**} und **R**_{**14**} für Phenyl oder einen anderen aromatischen Kohlenwasserstoff stehen, wobei diese Reste unsubstituiert oder mit C₁-C₁₈-Alkyl, C₃-C₁₈-Alkenyl, C₃-C₁₈-Alkinyl, C₁-C₁₈-Halogenalkyl, NO₂, OH, CN, OR₈, SR₈, C(O)R₉, C(O)OR₁₀ oder Halogen ein- oder mehrfach substituiert sind;
**R**_{**15**} C₁-C₁₈-Alkyl, Phenyl oder einen anderen aromatischen Kohlenwasserstoff darstellt, wobei die Reste Phenyl und aromatischer Kohlenwasserstoff unsubstituiert oder mit C₁-C₁₈-Alkyl, C₃-C₁₈-Alkenyl, C₃-C₁₈-Alkinyl, C₁-C₁₈-Halogenalkyl, NO₂, OH, CN, OR₈, SR₈, C(O)R₉, C(O)OR₁₀ oder Halogen ein- oder mehrfach substituiert sind, oder R₁₅ für einen Rest 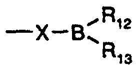 steht; und
**X** C₁-C₂₀-Alkylen, C₂-C₂₀-Alkylen, welches durch -O-, -S- oder NR₈ unterbrochen ist,
bedeutet oder X 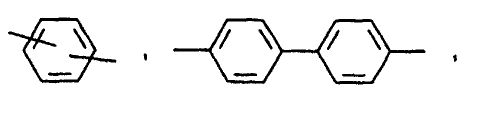 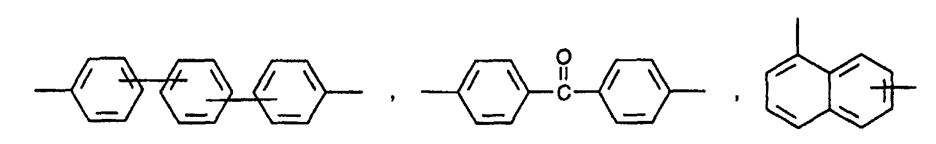 oder 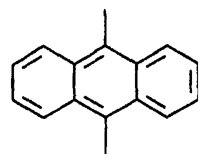 ist.

2. Verbindungen nach Anspruch 1, worin **R**_{**1**} Phenyl, Naphthyl, Pyrenyl, Thioxanthyl oder Phenothiazinyl bedeutet, wobei diese Reste unsubstituiert oder mit C₁-C₁₈-Alkyl, C₁-C₁₈-Halogenalkyl, NR₆R₇, CN, NO₂, SR₈ oder OR₈ ein- oder mehrfach substituiert sind.

3. Verbindungen nach Anspruch 1, worin **R**_{**2**}**, R**_{**3**} und **R**_{**4**} unabhängig voneinander Wasserstoff sind oder C₁-C₁₈-Alkyl, R₂ und R₃ und/oder R₄ und R₃ unabhängig voneinander eine C₂-C₁₂-Alkylenbrücke bilden; oder
R₂, R₃, R₄ mit dem Stickstoffatom, an welches sie gebunden sind, eine Gruppe der Strukturformeln (a), (b), (c), (d), (e), (f), (g), (h) oder eine Phosphazen-Base vom Typ P₁, P₂ oder P₄ bilden.

4. Verbindungen nach Anspruch 1, worin **R**_{**2**}**, R**_{**3**} und **R**_{**4**} C₁-C₁₈-Alkyl, mit dem Stickstoffatom eine Gruppe der Strukturformeln (a), (b), (c), (d) oder (e) bilden.

5. Verbindungen nach Anspruch 1, worin **R**_{**12**}**, R**_{**13**}**, R**_{**14**} Phenyl, Biphenyl, Naphthyl, Anthracyl oder Phenanthryl sind, wobei diese Reste unsubstituiert oder mit C₁-C₁₈-Alkyl, C₁-C₁₈-Halogenalkyl, NO₂, OH, CN, OR₈, oder Halogen ein- oder mehrfach substituiert sind, bedeuten und
R₁₅ C₁-C₁₈-Alkyl oder unsubstituiertes oder mit C₁-C₁₈-Alkyl, C₁-C₁₈-Halogenalkyl, NO₂, OH, CN, OR₈ oder Halogen ein- oder mehrfach substituiertes Phenyl bedeutet.

6. Verfahren zur Herstellung von Verbindungen der Formel I, **dadurch gekennzeichnet, dass** in einem ersten Schritt eine Stickstoffbase der Formel II
NR₂R₃R₄ (II)
mit einem α- Halogenalken der Formel III 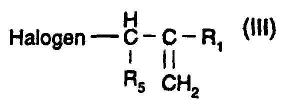 zu einer Verbindung der Formel IV 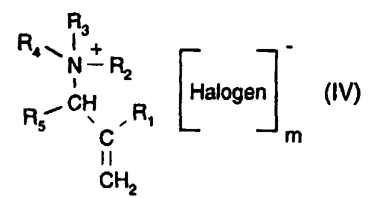 umgesetzt wird, und in einem zweiten Schritt die Verbindung der Formel IV mit einer Verbindung der Formel V 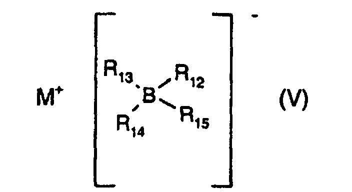 zur Verbindung der Formel I umgesetzt wird, worin
**Halogen** Brom oder lod bedeutet und
**M** Na, K oder Ammonium ist und die Reste
**R**_{**1**}**, R**_{**2**}**, R**_{**3**}**, R**_{**4**}**, R**_{**5**}**, R**_{**12**}**, R**_{**13**}**, R**_{**14**} und **R**_{**15**} die in Anspruch 1 angegebenen Bedeutungen haben.

7. Zusammensetzung, enthaltend
A) mindestens eine Verbindung der Formel (I) gemäss einem der Ansprüche 1-5,
B) mindestens eine organische Verbindung, welche zu einer basenkatalysierten Additions-oder Substitutionsreaktion befähigt ist.

8. Zusammensetzung nach Anspruch 7, worin die Komponente B) ein anionisch polymerisierbares oder vernetzbares organisches Material ist.

9. Zusammensetzung nach Anspruch 7, worin die Komponente B) eines der folgenden Systeme bedeutet:
a) ein Acrylatcopolymer mit Alkoxysilan- bzw. Alkoxysiloxan-Seitengruppen,
b) ein Zweikomponentensystem aus einem hydroxylgruppenhaltigen Polyacrylat und/oder Polyester und einem aliphatischen oder aromatischen Polyisocyanat,
c) ein Zweikomponentensystem aus einem funktionellen Polyacrylat und einem Polyepoxid, wobei das Polyacrylat Carboxyl- oder Anhydridgruppen enthält,
d) ein Zweikomponentensystem aus einem fluormodifizierten oder siliconmodifizierten hydroxylgruppenhaltigen Polyacrylat oder Polyester und einem aliphatischen oder aromatischen Polyisocyanat,
e) ein Zweikomponentensystem aus einem (Poly)ketimin und einem aliphatischen oder aromatischen Polyisocyanat,
f) ein Zweikomponentensystem aus einem (Poly)ketimin und einem ungesättigten Acrylatharz oder einem Acetoacetatharz oder Methyl-α-acrylamido-methylglykolat,
h) ein Zweikomponentensystem aus einem (Poly)oxazolidin und einem anhydridgruppenhaltigen Polyacrylat oder einem ungesättigten Acrylatharz oder einem Polyisocyanat,
i) ein Zweikomponentensystem aus einem epoxygruppenhaltigen Polyacrylat und einem carboxylgruppenhaltigen Polyacrylat,
I) ein Polymeres auf Basis von Allyl-glycidylether.
m) ein Zweikomponentensystem aus einem (Poly)alkohol und einem (Poly)isocyanat,
n) ein Zweikomponentensystem aus einer α,β-ethylenisch ungesättigten Carbonylverbindung und einer Verbindung mit aktivierten CH₂-Gruppen.

10. Zusammensetzung nach Anspruch 7, worin die Komponente B) eines der folgenden Systeme bedeutet:
b) ein Zweikomponentensystem aus einem hydroxylgruppenhaltigen Polyacrylat und/oder Polyester und einem aliphatischen oder aromatischen Polyisocyanat,
c) ein Zweikomponentensystem aus einem funktionellen Polyacrylat und einem Polyepoxid, wobei das Polyacrylat Carboxyl- oder Anhydridgruppen enthält,
i) ein Zweikomponentensystem aus einem epoxygruppenhaltigen Polyacrylat und einem carboxylgruppenhaltigen Polyacrylat,
m) ein Zweikomponentensystem aus einem (Poly)alkohol und einem (Poly)isocyanat,
n) ein Zweikomponentensystem aus einer α,β-ethylenisch ungesättigten Carbonylverbindung und einer Verbindung mit aktivierten CH₂-Gruppen.

11. Zusammensetzung nach Anspruch 7, worin die Komponente B ein Epoxidharz oder ein Gemisch aus verschiedenen Epoxidharzen ist.

12. Zusammensetzung nach Anspruch 7, worin die Komponente A) in einer Menge von 0,01 bis 10 Gew.-%, bezogen auf die Komponente B), enthalten ist.

13. Zusammensetzung nach Anspruch 7, welche zusätzlich einen Sensibilisator enthält, der aus der Gruppe Thioxanthone, Oxazine, Acridine, Phenazine oder Rhodamine ausgewählt ist.

14. Verfahren zur Durchführung basenkatalysierter Reaktionen, **dadurch gekennzeichnet dass** eine Zusammensetzung nach Anspruch 7 mit Licht einer Wellenlänge von 200 nm bis 650 nm bestrahlt wird.

15. Verfahren nach Anspruch 14, **dadurch gekennzeichnet, dass** während oder nach der Belichtung erwärmt wird.

16. Verwendung einer organischen Verbindung der Formel I gemäss einem der Ansprüche 1-5 als Photoinitiator für photochemisch induzierte basenkatalysierte Additions- oder Substitutionsreaktionen.

17. Verwendung einer organischen Verbindung der Formel I gemäss einem der Ansprüche 1-5, zur Herstellung von Beschichtungen, Formmassen oder photostrukturierten Schichten.

18. Beschichtetes Substrat, das auf mindestens einer Oberfläche mit einer Zusammensetzung nach Anspruch 7 beschichtet ist.

19. Polymerisierte oder vernetzte Zusammensetzung nach Anspruch 7.

## Claims

1. A compound of formula (I) 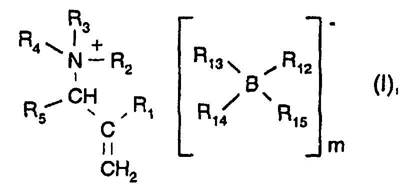 wherein
**m** is 1 or 2 and corresponds to the number of positive charges of the cation;
**R**_{**1**} is phenyl, naphthyl, phenanthryl, anthracyl, pyrenyl, 5,6,7,8-tetrahydro-2-naphthyl, 5,6,7,8-tetrahydro-1-naphthyl, thienyl, benzo[b]thienyl, naphtho[2,3-b]thienyl, thianthrenyl, dibenzofuryl, chromenyl, xanthenyl, thioxanthyl, phenoxathiinyl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, phthalazinyl, naphthyridinyl, quinoxalinyl, quinazolinyl, cinnolinyl, pteridinyl, carbazolyl, β-carbolinyl, phenanthridinyl, acridinyl, perimidinyl, phenanthrolinyl, phenazinyl, isothiazolyl, phenothiazinyl, isoxazolyl, furazanyl, terphenyl, stilbenyl, fluorenyl or phenoxazinyl, those radicals being unsubstituted or mono- or poly-substituted by C₁-C₁₈alkyl, C₃-C₁₈alkenyl, C₃-C₁₈alkynyl, C₁-C₁₈haloalkyl, NO₂, NR₆R₇, N₃, OH, CN, OR₈, SR₈, C(O)R₉, C(O)OR₁₀ or by halogen, or R₁ is a radical of formula A or B 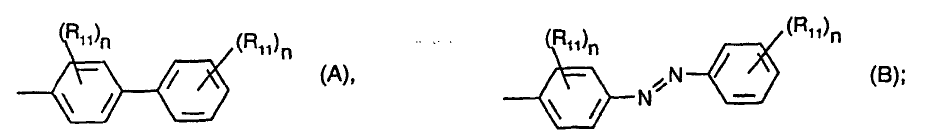
**R**_{**2**}**, R**_{**3**} and **R**_{**4**} are each independently of the others hydrogen, C₁-C₁₈alkyl, C₃-C₁₈alkenyl, C₃-C₁₈alkynyl or phenyl or, independently of one another, R₂ and R₃ and/or R₄ and R₃ form a C₂-C₁₂alkylene bridge; or R₂, R₃, R₄, together with the nitrogen atom to which they are bonded, form a phosphazene base of the type P₁, P₂, P₄ or a group of the structural formula (a), (b), (c), (d), (e), (f) or (g) 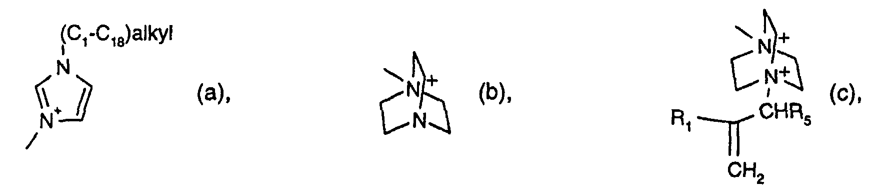 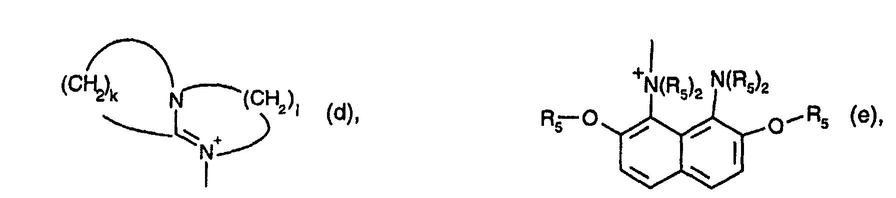 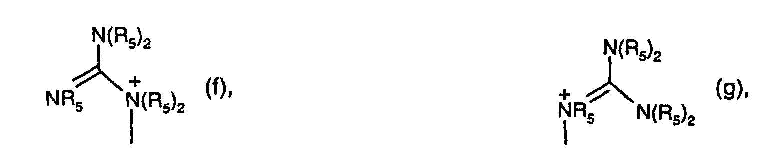 wherein
**k** and **I** are each independently of the other a number from 2 to 12;
**R**_{**5**}**, R**_{**6**}**, R**_{**7**}**, R**_{**8**}**, R**_{**9**} and **R**_{**10**} are each independently of the others hydrogen or C₁-C₁₈alkyl;
**R**_{**11**} is C₁-C₁₈alkyl, C₂-C₁₈alkenyl, C₂-C₁₈alkynyl, C₁-C₁₈haloalkyl, NO₂, NR₆R₇, OH, CN, OR₈, SR₈, C(O)R₉, C(O)OR₁₀ or halogen; and
**n** is 0 or a number 1, 2 or 3;
**R**_{**12**}**, R**_{**13**} and **R**_{**14**} are phenyl or another aromatic hydrocarbon, those radicals being unsubstituted or mono- or poly-substituted by C₁-C₁₈alkyl, C₃-C₁₈alkenyl, C₃-C₁₈alkynyl, C₁-C₁₈haloalkyl, NO₂, OH, CN, OR₈, SR₈, C(O)R₉, C(O)OR₁₀ or by halogen;
**R**_{**15**} is C₁-C₁₈alkyl, phenyl or another aromatic hydrocarbon, the phenyl and aromatic hydrocarbon radicals being unsubstituted or mono- or poly-substituted by C₁-C₁₈alkyl, C₃-C₁₈-alkenyl, C₃-C₁₈alkynyl, C₁-C₁₈haloalkyl, NO₂, OH, CN, OR₈, SR₈, C(O)R₉, C(O)OR₁₀ or by halogen, or R₁₅ is a radical 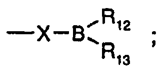 and
**X** is C₁-C₂₀alkylene, C₂-C₂₀alkylene interrupted by -O -S- or NR₈, or X is

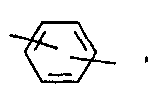

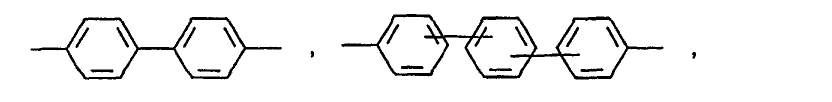

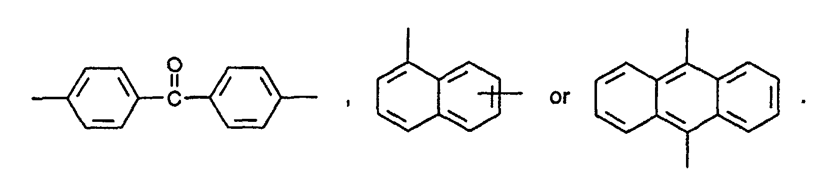

2. A compound according to claim 1, wherein **R**_{**1**} is phenyl, naphthyl, pyrenyl, thioxanthyl or phenothiazinyl, those radicals being unsubstituted or mono- or poly-substituted by C₁-C₁₈-alkyl, C₁-C₁₈haloalkyl, NR₆R₇, CN, NO₂, SR₈ or by OR₈.

3. A compound according to claim 1, wherein **R**_{**2**}**, R**_{**3**} and **R**_{**4**} are each independently of the others hydrogen or C₁-C₁₈alkyl, R₂ and R₃ and/or R₄ and R₃, independently of one another, form a C₂-C₁₂alkylene bridge; or
R₂, R₃, R₄, together with the nitrogen atom to which they are bonded, form a group of the structural formula (a), (b), (c), (d), (e), (f), (g) or (h) or a phosphazene base of the type P₁, P₂ or P₄.

4. A compound according to claim 1, wherein **R**_{**2**}**, R**_{**3**} and **R**_{**4**} C₁-C₁₈alkyl, together with the nitrogen atom, form a group of the structural formula (a), (b), (c), (d) or (e).

5. A compound according to claim 1, wherein **R**_{**12**}**, R**_{**13**} and **R**_{**14**} are phenyl, biphenyl, naphthyl, anthracyl or phenanthryl, those radicals being unsubstituted or mono- or poly-substituted by C₁-C₁₈alkyl, C₁-C₁₈haloalkyl, NO₂, OH, CN, OR₈ or by halogen, and R₁₅ is C₁-C₁₈alkyl, unsubstituted phenyl or phenyl mono- or poly-substituted by C₁-C₁₈alkyl, C₁-C₁₈haloalkyl, NO₂, OH, CN, OR₈ or by halogen.

6. A process for the preparation of a compound of formula I wherein, in a first step, a nitrogen base of formula II
NR₂R₃R₄ (II)
is reacted with an α-haloalkene of formula III 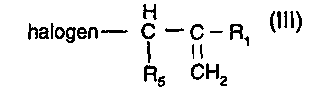 to form a compound of formula IV 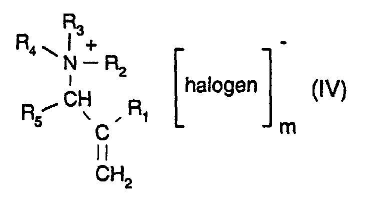 and, in a second step, the compound of formula IV is reacted with a compound of formula V 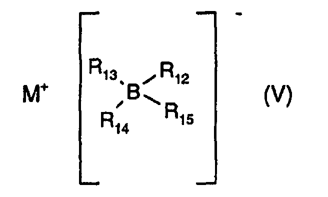 to form a compound of formula I wherein
**halogen** is bromine or iodine and
**M** is sodium, potassium or ammonium, and the radicals
**R**_{**1**}**, R**_{**2**}**, R**_{**3**}**, R**_{**4**}**, R**_{**5**}**, R**_{**12**}**, R**_{**13**}**, R**_{**14**} and **R**_{**15**} are as defined in claim 1.

7. A composition comprising
A) at least one compound of formula (I) according to anyone of claims 1-5
B) at least one organic compound that is capable of a base-catalysed addition or substitution reaction.

8. A composition according to claim 7, wherein component B) is an anionically polymerisable or crosslinkable organic material.

9. A composition according to claim 7, wherein component B) is one of the following systems:
a) an acrylate copolymer having alkoxysilane or alkoxysiloxane side groups;
b) a two-component system consisting of a hydroxy-group-containing polyacrylate and/or polyester and an aliphatic or aromatic polyisocyanate;
c) a two-component system consisting of a functional polyacrylate and a polyepoxide, the polyacrylate containing carboxy or anhydride groups;
d) a two-component system consisting of a fluorine-modified or silicone-modified hydroxy-group-containing polyacrylate or polyester and an aliphatic or aromatic polyisocyanate;
e) a two-component system consisting of a (poly)ketimine and an aliphatic or aromatic polyisocyanate;
f) a two-component system consisting of a (poly)ketimine and an unsaturated acrylate resin or an acetoacetate resin or methyl-α-acrylamido-methylglycolate;
h) a two-component system consisting of a (poly)oxazolidine and an anhydride-group-containing polyacrylate or an unsaturated acrylate resin or a polyisocyanate;
i) a two-component system consisting of an epoxy group-containing polyacrylate and a carboxy-group-containing polyacrylate;
I) a polymer based on an allyl-glycidyl ether;
m) a two-component system consisting of a (poly)alcohol and a (poly)isocyanate;
n) a two-component system consisting of an α,β-ethylenically unsaturated carbonyl compound and a compound having activated CH₂ groups.

10. A composition according to claim 7, wherein component B) is one of the following systems:
b) a two-component system consisting of a hydroxy-group-containing polyacrylate and/or polyester and an aliphatic or aromatic polyisocyanate;
c) a two-component system consisting of a functional polyacrylate and a polyepoxide, the polyacrylate containing carboxy or anhydride groups;
i) a two-component system consisting of an epoxy-group-containing polyacrylate and a carboxy-group-containing polyacrylate;
m) a two-component system consisting of a (poly)alcohol and a (poly)isocyanate;
n) a two-component system consisting of an α,β-ethylenically unsaturated carbonyl compound and a compound having activated CH₂ groups.

11. A composition according to claim 7, wherein component B is an epoxy resin or a mixture of different epoxy resins.

12. A composition according to claim 7, wherein component A) is present in an amount of from 0.01 to 10 % by weight based on component B).

13. A composition according to claim 7 that in addition comprises a sensitiser selected from the thioxanthone, oxazine, acridine, phenazine and rhodamine group.

14. A method of carrying out a base-catalysed reaction, which comprises irradiating a composition according to claim 7 with light of a wavelength of from 200 nm to 650 nm.

15. A method according to claim 14, wherein heating is carried out during or after the irradiation.

16. The use of an organic compound of formula I according to anyone of claims 1-5, as a photoinitiator for photochemically induced base-catalysed addition or substitution reactions.

17. The use of an organic compound of formula I according to anyone of claims 1-5, in the preparation of coatings, moulding materials or photostructured layers.

18. A coated substrate that is coated on at least one surface with a composition according to claim 7.

19. A polymerised or crosslinked composition according to claim 7.

## Revendications

1. Composés de formule (I) 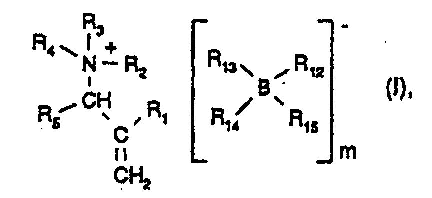
**m** vaut 1 ou 2 et correspond au nombre de charges positives du cation ;
**R**_{**1**} représente des groupes phényle, naphtyle, phénanthryle, anthracyle, pyrényle, 5,6,7,8-tétrahydro-2-naphtyle, 5,6,7, 8-tétrahydro-1-naphtyle, thiényle, benzo[b]thiényle, naphto[2,3-b]thiényle, thianthrényle ; dibenzofuryle, chroményle, xanthényle, thioxanthyle, phénoxathiinyle, pyrrolyle, imidazolyle, pyrazolyle, pyrazinyle, pyrimidinyle, pyridazinyle, indolizinyle, isoindolyle, indolyle, indazolyle, purinyle, quinolizinyle, isochinolyle, quinolyle, phthalazinyle, naphtyridinyle, quinoxalinyle, quinazolinyle, cinnolinyle, ptéridinyle, carbazolyle, β-carbolinyle, phénanthridinyle, acridinyle, périmidinyle, phénanthrolinyle, phénazinyle, isothiazolyle, phénothiazinyle, isoxazolyle, furazanyle, terphényle, stilbényle, fluorényle ou phénoxazinyle, ces restes étant non substitués ou substitués une ou plusieurs fois par des substituants alkyle en C₁-C₁₈, alcényle en C₃-C₁₈, alcynyle en C₃-C₁₈, haloalkyle en C₁-C₁₈, NO₂, NR₆R₇, N₃, OH, CN, OR₈, SR₈, C(O)R₉, C(O)OR₁₀ ou halogène, ou R₁ représente un reste de formules A ou B 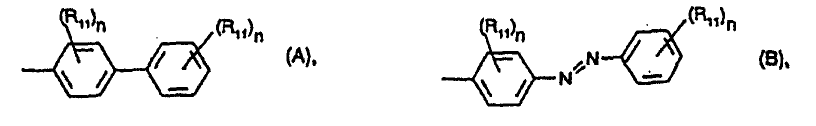
**R**_{**2**}**, R**_{**3**} et **R**_{**4**} représentent, indépendamment l'un de l'autre, un atome d'hydrogène, des groupes alkyle en C₁-C₁₈, alcényle en C₃-C₁₈, alcynyle en C₃-C₁₈ ou phényle ou R₂ et R₃ et/ou R₄ et R₃ forment, indépendamment l'un de l'autre, un pont alkylène en C₂-C₁₂ ; ou R₂, R₃, R₄ forment, conjointement avec l'atome d'azote auquel ils sont liés, une base phosphazane de type P₁, P₂, P₄ ou un groupe de formules développées (a), (b), (c), (d), (e), (f) ou (g), 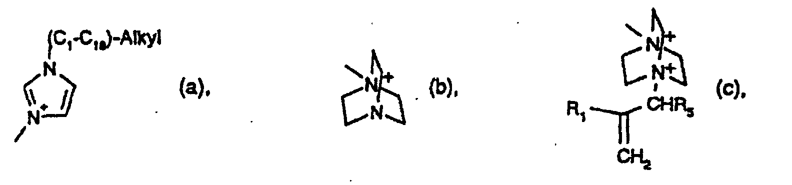 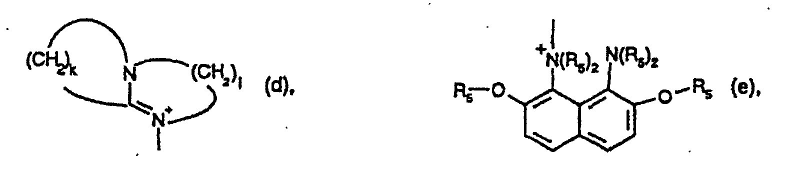 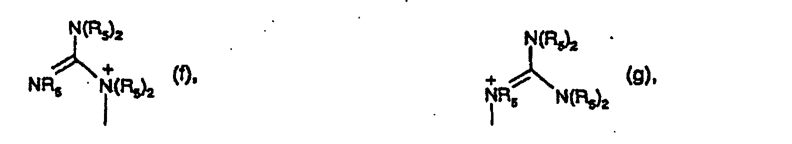
où
**k** et **l** sont, indépendamment l'un de l'autre, un nombre de 2 à 12;
**R**_{**5**}**, R**_{**6**}**, R**_{**7**}**, R**_{**8**}**, R**_{**9**} et **R**_{**10**} représentent, indépendamment l'un de l'autre, un atomes d'hydrogène ou un groupe alkyle en C₁-C₁₈, ;
**R**_{**11**} représente des groupes alkyle en C₁-C₁₈, alcényle en C₂-C₁₈, alcynyle en C₂-C₁₈, haloalkyle en C₁-C₁₈, NO₂, NR₆R₇, OH, CN, OR₈, SR₈, C(O)R₉, C(O)R₁₀ ou halogène ; et
**n** vaut zéro ou un nombre 1, 2 ou 3 ;
**R**_{**12**}**, R**_{**13**} et **R**_{**14**} représentent un groupe phényle ou un autre hydrocarbure aromatique, ces restes étant non substitués ou substitués une ou plusieurs fois par des substituants alkyle en C₁-C₁₈, alcényle en C₃-C₁₈, alcynyle en C₃-C₁₈, haloalkyle en C₁-C₁₈, NO₂, OH, CN, OR₈, SR₈, C(O)R₉, C(O)R₁₀ ou halogène ;
**R**_{**15**} représente un groupe alkyle en C₁-C₁₈, un groupe phényle ou un hydrocarbure aromatique différent, les restes phényle et hydrocarbure aromatique sont non substitués ou substitués une ou plusieurs fois par des substituants alkyle en C₁-C₁₈, alcényle en C₃-C₁₈, alcynyle en C₃-C₁₈, haloalkyle en C₁-C₁₈, NO₂, OH, CN, OR₈, SR₈, C(O)R₉, C(O)R₁₀ ou halogène, ou R₁₅ représente un reste 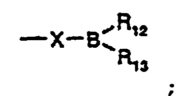 et
X représente des groupes alkylène en C₁-C₂₀, alkylène en C₂-C_{20,} qui est interrompu par des groupes -O-, -S- ou NR₈, ou X représente des groupes 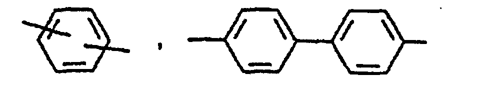 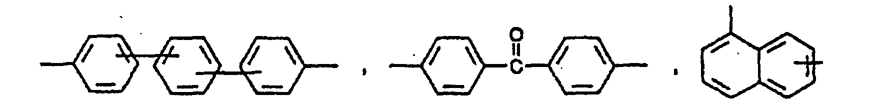
ou

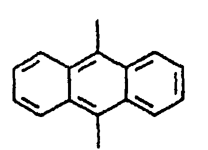

2. Composés selon la revendication 1, où **R**_{**1**} représente des groupes phényle, naphtyle, pyrényle, thioxanthyle ou phénothiazinyle, ces restes étant non substitués ou substitués une ou plusieurs fois par des substituants alkyle en C₁-C₁₈, haloalkyle en C₁-C₁₈, NR₆R₇, CN, NO₂, SR₈ ou OR₈.

3. Composés selon la revendication 1, où **R**_{**2**}**, R**_{**3**} et **R**_{**4**} représentent, indépendamment l'un de l'autre, des atomes d'hydrogène ou des groupes alkyle en C₁-C₁₈, R₂ et R₃ et/ou R₄ et R₃ forment, indépendamment l'un de l'autre, un pont alkylène en C₂-C₁₂ ; ou
R₂, R₃, R₄ forment, conjointement avec l'atome d'azote auquel ils sont liés, un groupe de formules développées (a), (b), (c), (d), (e), (f), (g), (h) ou une base phosphazane de type P₁, P₂ ou P₄.

4. Composés selon la revendication 1, où **R**_{**2**}**, R**_{**3**} et **R**_{**4**} représentent un groupe alkyle en C₁-C₁₈, forment avec l'atome d'azote auquel ils sont liés, un groupe de formules développées (a), (b), (c), (d) ou (e).

5. Composés selon la revendication 1, où **R**_{**12**}**, R**_{**13**}**, R**_{**14**} représentent des groupes phényle, biphényle, naphtyle, anthracyle ou phénanthryle, ces restes sont non substitués ou substitués une ou plusieurs fois par des substituants alkyle en C₁-C₁₈, haloalkyle en C₁-C₁₈, NO₂, OH, CN, OR₈ ou halogène, et
R₁₅ représente un groupe alkyle en C₁-C₁₈ ou un groupe phényle non substitué ou substitué une ou plusieurs fois par des substituants alkyle en C₁-C₁₈, haloalkyle en C₁-C₁₈, NO₂, OH, CN, OR₈ ou halogène.

6. Procédé pour la préparation de composés de formule I, **caractérisé en ce que**, dans une première étape, on fait réagir une base azotée de formule (II)
NR₂R₃R₄ (II)
sur un α-halogénoalcène de formule (III) 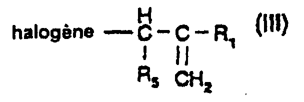 pour obtenir un composé de formule IV 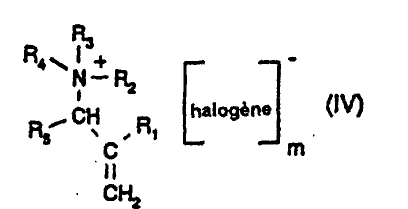 et dans une seconde étape, on fait réagir le composé de formule IV sur un composé de formule (V) 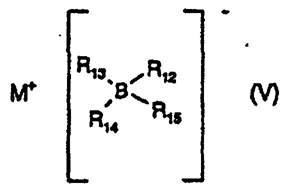 pour obtenir un composé de formule I, où **halogène** représente un atome de brome ou d'iode et **M** représente Na, K ou ammonium et les restes **R**_{**1**}**, R**_{**2**}**, R**_{**3**}**, R**_{**4**}**, R**_{**5**}**, R**_{**12**}**, R**_{**13**}**, R**_{**14**} et **R**_{**15**} possèdent les significations données à la revendication 1.

7. Composition contenant
A) au moins un composé de formule (I) selon l'une des revendications 1 à 5,
B) au moins un composé organique, qui est capable de réagir dans une réaction d'addition ou de substitution catalysée par des bases.

8. Composition selon la revendication 7, où le composant B) est une matière organique polymérisable par polymérisation anionique ou réticulable.

9. Composition selon la revendication 7, où le composant B) représente un des systèmes suivants :
a) un polymère acrylate présentant des groupes latéraux alkoxysilanes ou alkoxysiloxanes,
b) un système à deux composants constitué par un polyacrylate et/ou polyester présentant des groupes hydroxyle et par un polyisocyanate aliphatique ou aromatique,
c) un système à deux composants constitué par un polyacrylate fonctionnel et par un polyépoxyde, le polyacrylate présentant des groupes carboxyles ou anhydrido,
d) un système à deux composants constitué par un polyacrylate ou polyester présentant des groupes hydroxyle, modifié par du fluor ou du silicium, ou par un polyisocyanate aliphatique ou aromatique,
e) un système à deux composants constitué par une (poly)cétimine et par un polyisocyanate aliphatique ou aromatique,
f) un système à deux composants constitué par une (poly)cétimine et une résine acrylique insaturée ou par une résine acétoacétate ou un glycolate de méthyl-α-acrylamido-méthyle,
h) un système à deux composants constitué par un (poly)-oxazolidine et un polyacrylate présentant des groupes anhydrido ou par une résine acrylique insaturée ou un polyisocyanate aliphatique ou aromatique,
i) un système à deux composants constitué par un polyacrylate présentant des groupes époxydes et un polyacrylate présentant des groupes carboxyles,
l) un polymère à base d'éther allyl-glycidylique,
m) un système à deux composants constitué par un (poly)alcool et un (poly)isocyanate,
n) un système à deux composants constitué par un composé carbonyle à insaturation éthylénique en α,β et un composé avec des groupes CH₂ activés.

10. Composition selon la revendication 7, où le composant B) représente un des systèmes suivants :
b) un système à deux composants constitué par un polyacrylate et/ou polyester présentant des groupes hydroxyle et par un polyisocyanate aliphatique ou aromatique,
c) un système à deux composants constitué par un polyacrylate fonctionnel et un polyépoxyde, le polyacrylate présentant des groupes carboxyles ou anhydrido,
i) un système à deux composants constitué par un polyacrylate présentant des groupes époxydes et un polyacrylate présentant des groupes carboxyles,
m) un système à deux composants constitué par un (poly)alcool et un (poly)isocyanate,
n) un système à deux composants constitué par un composé carbonylé à insaturation éthylénique en α,β et un composé avec des groupes CH₂ activés.

11. Composition selon la revendication 7, où le composant B) est une résine époxyde ou un mélange de différentes résines époxydes.

12. Composition selon la revendication 7, où le composant A) est présent dans une quantité de 0,01 à 10 % en poids, par rapport au composant B).

13. Composition selon la revendication 7, qui contient de plus un sensibilisant, qui est pris dans le groupe comprenant les thioxanthones, les oxazines, les acridines, les phénazines ou les rhodamines.

14. Procédé pour la mise en oeuvre de réactions catalysées par des bases, **caractérisé** en qu'on irradie une composition selon la revendication 7 par une lumière d'une longueur d'onde de 200 nm à 600 nm.

15. Procédé selon la revendication 14, **caractérisé en ce qu'**on chauffe au cours de l'irradiation.

16. Utilisation d'un composé organique de formule I selon une ou plusieurs des revendications 1 à 5 en tant que photoamorceur pour des réactions d'addition ou de substitution induites par voie photochimique, catalysées par des bases.

17. Utilisation d'un composé organique de formule I selon une ou plusieurs des revendications 1 à 5 pour la préparation de revêtements, de matières à mouler ou de couches photostructurées.

18. Substrat revêtu, qui est revêtu sur au moins une face d'une composition selon la revendication 7.

19. Composition polymérisée ou réticulée selon la revendication 7.
